# FASCICULE DE BREVET EUROPEEN

(11) **EP 0 819 136 B1**
(45) Date de publication et mention de la délivrance du brevet: **30.01.2002**
(21) Numéro de dépôt: 96912079.9
(22) Date de dépôt: 05.04.1996
(51) Int. Cl.: C07K 9/00, A61K 47/18

(54) **DERIVES ET CONJUGUES DU MDP PRESENTANT UNE ACTIVITE STIMULATRICE DE LA FONCTION HEMATOPOIETIQUE ET COMPOSITION LES CONTENANT**
DERIVATE UND KONJUGETE VON MDP, EINE VON HÄMATOPOITISCHER FUNKTION STIMULIERENDE WIRKUNG UND DEREN ENTHALTENDE ZUSAMMENSETZUNGEN
MDP DERIVATIVES AND CONJUGATES HAVING HAEMATOPOIETIC FUNCTION STIMULATING ACTIVITY, AND COMPOSITIONS CONTAINING SAME

(30) Priorité: 07.04.1995 FR 9504194
(43) Date de publication de la demande: 21.01.1998
(73) Titulaire: VACSYN S.A., 75015 Paris (FR)
(72) Inventeur: BAHR, Georges, F-92800 Puteaux (FR); LEFRANCIER, Pierre, F-91190 Gif-sur-Yvette (FR); CHEDID, Louis, F-75015 Paris (FR)
(74) Mandataire: Becker, Philippe
(86) Numéro de dépôt international: FR9600527
(87) Numéro de publication internationale: WO9631533

(56) Documents cités:
- EP-A- 0 089 290
- EP-A- 0 099 578
- EP-A- 0 192 609
- WO-A-95/19777
- FR-A- 2 343 483
- INT. CONGR. SER. EXCERPTA MEDICA, vol. 563, 1982, pages 137-240, XP002008784
- CARBOHYDRATE RESEARCH, vol. 81, no. 1, 1981, AMSTERDAM NL, pages 173-176, XP002008785 T D J HALLS ET AL.: "The anomeric configuration of the immunostimulant N-acetylmuramoyl-dipeptide and some of its derivatives"

## Description

L'invention est relative à une composition pharmaceutique capable de stimuler la fonction hématopoïétique de manière à mobiliser et faire apparaître dans la circulation des taux extrêmement élevés de cellules souches provenant de la moelle osseuse. Cette invention permet de proposer :
1) des thérapies antimyélotoxiques nouvelles ;
2) des procédés nouveaux de récolte de cellules souches de la moelle osseuse permettant de remplacer les biopsies de moelle en vue :
   a) de greffes autologues ou homologues,
   b) d'utilisation dans les techniques de thérapie génique.

Ladite composition pharmaceutique comprend au moins un dérivé de MDP de formule générale (I) ci-dessous, et dont un exemple particulièrement performant pour l'objet de l'invention est le Muradimétide de formule Nac-Mur-L-Ala-D-Glu-[OCH3]-OCH3 ou MDPA-diméthyl ester.

Les muramyl peptides et leurs dérivés esters sont une famille de molécules qui ont été décrites dans de nombreuses publications et de nombreux brevets depuis la première description du N-Acétyl-Muramyl-L-Alanyl-D-isoGlutamine (MDP pour muramyl dipeptide).
Brièvement, ils sont capables d'exercer une grande variété d'activités biologiques incluant une activité immuno-adjuvante, une activité anti-infectieuse, une activité anti-tumorale, une activité anti-allergique et une régulation de la sécrétion des cytokines. Cependant, un certain nombre de ces muramyl peptides, s'ils peuvent présenter des activités biologiques tout à fait prometteuses, présentent souvent des effets secondaires ou des inconvénients qui en empêchent leur utilisation à titre de médicament, notamment des effets toxiques ou une difficulté de formulation du fait de leur faible solubilité dans des solutions aqueuses.

Ils présentent en outre une grande variabilité dans leur spectre d'action.

Très récemment, il a été montré que les muramyl peptides diesters de formule générale (I) ci-dessous et, en particulier, le Muradimétide possèdent la propriété particulière d'être immunostimulants ce qui présente à la fois des avantages d'efficacité, de formulation, et de non toxicité, cumul d'avantages qui n'avait pas été observé pour d'autres dérivés du MDP ; en particulier, un immunomodulateur actif par administration par voie externe, notamment par voie orale, par aérosol, peut permettre d'envisager une utilisation plus large et plus souple de cet immunostimulant; l'avantage de bonne tolérance se retrouve également quand ce dérivé est administré par voie générale.

L'essentiel des propriétés déjà décrites sur les dérivés de MDP est lié à un renforcement du système immunitaire humoral ou cellulaire.

Par exemple, FR A 234 348 divulgue des compositions de muramyl peptide pour leur utilisation en tant que compositions médicales adjuvantes.

EP 0 099 578 divulgue des composés similaires à certains de ceux qui sont décrits dans la présente description, présentés comme ayant une activité anti-virale.

EP 0 089 290 enseigne des haptènes portant un site antigénique qui sont conjugués à un muramyl peptide pour être utilisés en tant qu'immunogènes.

EP 0 192 609 divulgue plusieurs dérivés de muramyl peptide ayant des activités anti-virales et anti-infectieuses.

EP 0 056 992 enseigne plusieurs dérivés de muramyl peptide, parmi lesquels MTP-PE en tant qu'immunomodulateurs et immunostimulants.

Carbohydrate Research, 81, 173-176 (1980) divulgue plusieurs dérivés de muramyl peptide qui sont immunostimulants et leurs configurations aromériques.

Et Congr. Exerpta Medica, vol. 81, 237-240 (1982) enseigne plusieurs dérivés muramyl peptides parmi lesquels la muroctasine pour améliorer la résistance non-spécifique contre des infections bactériennes.

WO 95/19777 divulgue l'utilisation de plusieurs muramyl peptides, sous forme orale, en combinaison avec l'AZT en tant qu'agents immunostimulants.

De façon surprenante, la présente invention résulte de la découverte selon laquelle certains types de dérivés du MDP représentés ci-après par les formules (I), (II), (III) en association le cas échéant avec un composé de formule (IV) ou les conjugués de formules (V) et (VI) sont capables d'augmenter le taux d'éléments figurés normaux et immatures du sang et en particulier de neutrophiles dans le sang circulant, notamment après administrations de doses d'AZT susceptibles de diminuer considérablement le taux des leucocytes circulants. En effet, après administration de la composition pharmaceutique, on observe dans la circulation une augmentation de leucocytes normaux accompagnée d'une très forte augmentation de myélocytes/promyélocytes suivie d'une augmentation des neutrophiles et plus tardivement des lymphocytes. De façon tout à fait remarquable, cet effet s'observe très fortement même après un traitement combiné faisant intervenir des doses hautement myélotoxiques d'AZT.

Toutes les cellules sanguines dérivent de cellules souches communes dont l'origine est la moelle osseuse. Avant d'atteindre leur stade final de différenciation, ces cellules passent par différentes étapes : cellules souches pluripotentes pouvant se différencier soit en prolymphocytes T ou B, soit en cellules souches des lignées érythrocytaire, myélocytaire, granulocytaire et myélocaryocytaire. A ces différentes étapes intermédiaires, elles ne sont pas discernables morphologiquement et sont désignées par les termes promyélocyte/myélocyte pour les plus jeunes et métamyélocytes pour celles les plus proches de la différentiation.

Dans la suite du texte, l'ensemble de ces cellules non différenciées sera désigné par CSH pour « cellules souches hématopoïétiques ».

A l'état normal, le taux des CSH est de l'ordre de 1% des cellules blanches présentes dans le sang.

II est intéressant d'activer l'hématopoïèse et de mobiliser des CSH dans les trois cas suivants:
1) pour contrecarrer les effets myélotoxiques de certaines drogues afin de maintenir un taux normal de cellules sanguines (en particulier de neutrophiles),
2) pour remplacer des biopsies de moelle osseuse par la récolte de taux élevés de CSH dans le sang circulant en vue de greffe homologue ou autologue de cellules de moelle,
3) pour obtenir à partir du sang circulant un taux élevé de CSH afin de les utiliser comme vecteurs pour la réalisation de certaines thérapies géniques.

1) Traitement anti-myélotoxique.
   Certains traitements ou médicaments entraînent des effets myélotoxiques, tels les chimiothérapies du cancer ou du SIDA, des immunothérapies ou des radiothérapies, conduisant à un dysfonctionnement de la fonction hématopoïétique et augmentant ainsi dramatiquement les risques d'infections intercurrentes. La composition pharmaceutique de l'invention permet de s'opposer à la diminution des cellules du système immunitaire, résultant de cette myélotoxicité et en particulier des neutrophiles.
   En outre, certains traitements qui utilisent l'immunothérapie, notamment par les cytokines, peuvent également entraîner un effet cytotoxique pour lequel les compositions de l'invention peuvent jouer un rôle d'antagoniste.
2) Obtention de cellules permettant de pratiquer des greffes de moelle osseuse.
   Les traitements anticancéreux par chimiothérapie sévère ou par radiothérapie entraînent une destruction des cellules du sang et des cellules du système immunitaire. Dans ces cas, on reconstitue le système hématopoïétique par greffe de cellules de la moelle osseuse. On pratique des greffes autologues quand le cancer affecte des cellules autres que celles de la moelle et dans ce cas les prélèvements se font chez le patient lui-même avant le traitement destructeur. On pratique des greffes homologues (c'est-à-dire en utilisant des cellules provenant d'un donneur compatible) quand les cellules de la moelle sont elles-mêmes cancéreuses.
   Dans les deux cas, il est extrêmement intéressant de pouvoir enrichir la moelle osseuse en cellules souches mais surtout de pouvoir en obtenir dans le sang circulant en nombre suffisamment élevé pour faciliter leur utilisation comme greffons. En effet à l'heure actuelle, l'utilisation du sang pour la préparation de greffons enrichis en CSH demande des prélèvements nombreux et importants et donc étalés sur une longue période ; de plus il faut mettre en pratique une préparation pénible pour le malade et pour le donneur car l'enrichissement en CSH se fait par administration de cytokines coûteuses et parfois mal tolérées (GM-CSF) suivies de prélèvements sanguins nombreux et importants, l'ensemble de traitements nécessitant à chaque fois une hospitalisation. La composition pharmaceutique de l'invention permet d'obtenir le matériel nécessaire aux greffes de moelle chez des sujets ambulatoires et après quelques jours de traitement seulement, car elle est capable d'induire dans le sang, après environ 4 jours de traitement, une multiplication par 200 du nombre de CSH.
3) Obtention de cellules souches aisément purifiables utilisables pour la thérapie génique.

L'utilisation de certaines formes de techniques de thérapie génique dépendra de la possibilité d'obtenir des CSH en grande quantité dans le sang circulant. Cette obtention est possible grâce à la composition pharmaceutique de l'invention permettant d'obtenir un pourcentage extrêmement important de CSH dans le sang, alors que ces cellules sont très difficiles à purifier à partir de la moelle osseuse.

Les expériences précliniques et les essais cliniques de thérapie génique ont indiqué qu'il était nécessaire d'utiliser des cellules souches qui ont une longue durée de vie, un haut pouvoir d'expression et qui se répartissent dans tout l'organisme. Il est très généralement admis que les CSH sont des vecteurs de gènes particulièrement intéressants soit pour contrecarrer des déficits immunitaires telle la maladie de Gaucher ou les cas de déficits immunitaires profonds (SCID pour Severe Combined Immune Deficiencies), soit pour s'opposer au développement de cancers tels que myélome, leucémie, cancer du sein ou de l'ovaire, etc..., soit pour induire une résistance aux maladies infectieuses particulièrement le SIDA.

Pour répondre à ces objectifs, il est donc indispensable de disposer d'un nombre important de CSH aisément purifiables à partir du sang circulant par les techniques habituelles de séparation cellulaire et c'est justement ce que permet la composition de l'invention ou les médicaments la contenant.

La composition de l'invention permet d'envisager le développement de l'utilisation des cellules du sang circulant pour reconstituer la fonction hématopoïétique après destruction cellulaire grâce à une multiplication, qui peut atteindre un facteur 200, du nombre de CSH circulantes, alors qu'en l'absence de traitement, on ne peut en déceler plus de 1%.

Cet effet sur les CSH apparait quel que soit le stade de différenciation desdites cellules souches comme le montre l'exemple 7 ci-après.

Il résulte de cette découverte des nouvelles propriétés de certains dérivés du MDP, des propriétés intéressantes de compositions pharmaceutiques destinées à prévenir ou traiter l'effet myélotoxique de certains traitements ou maladies et/ou stimuler la fonction hématopoïétique. La présente invention est également relative à l'utilisation d'un composé dans un procédé pour la fabrication d'une composition pharmaceutique destinée à induire la stimulation de cellules souches hématopoïétiques et à les mobiliser dans le sang circulant caractérisée en ce qu'elle contient comme principe actif au moins un composé de la formule suivante (I) : 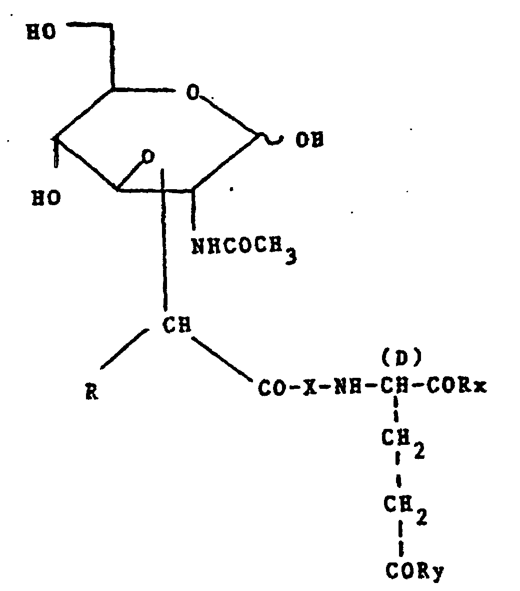 avec
- R =: H ou CH₃
- X =: L-ALa ou L-Thr
- Rx =: NH₂ out O(CH₂)ₓH avec x = 1 à 4
- Ry =: OH (sauf si Rx = NH₂) ou
O(CH₂)ₓH avec x = 1 à 4
ou la muroctasine de formule (II) suivante :
Nac-Mur-L.Ala-D-isoGln-N^{Σ} stearoyl-L-Lys. 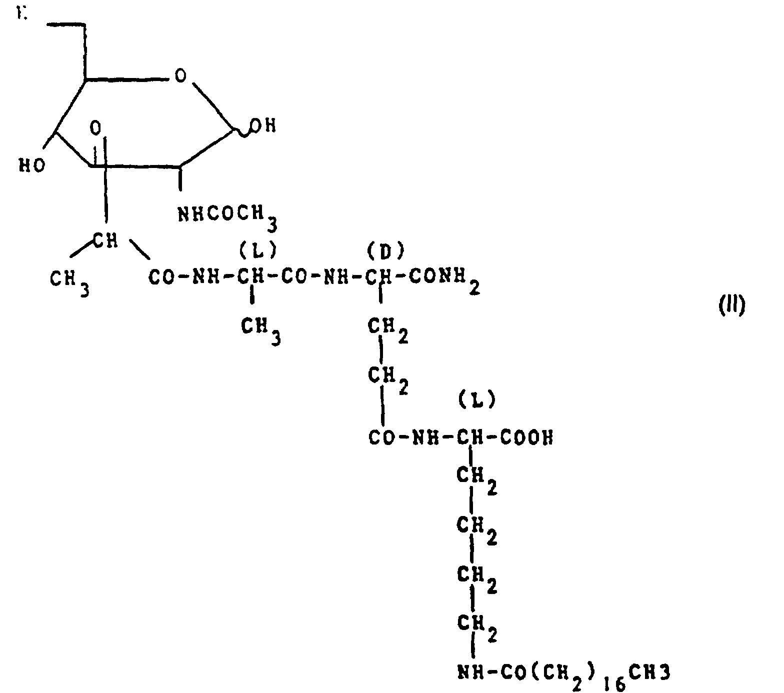 ou le MTP-PE de formule (III) suivante :
Nac-Mur-L-Ala-D-isoGln-L-alanyl-2 (1', 2'-dipalmitoyl-sn-glycero-3'-phosphoryl)-ethylamide-mono-sodium)

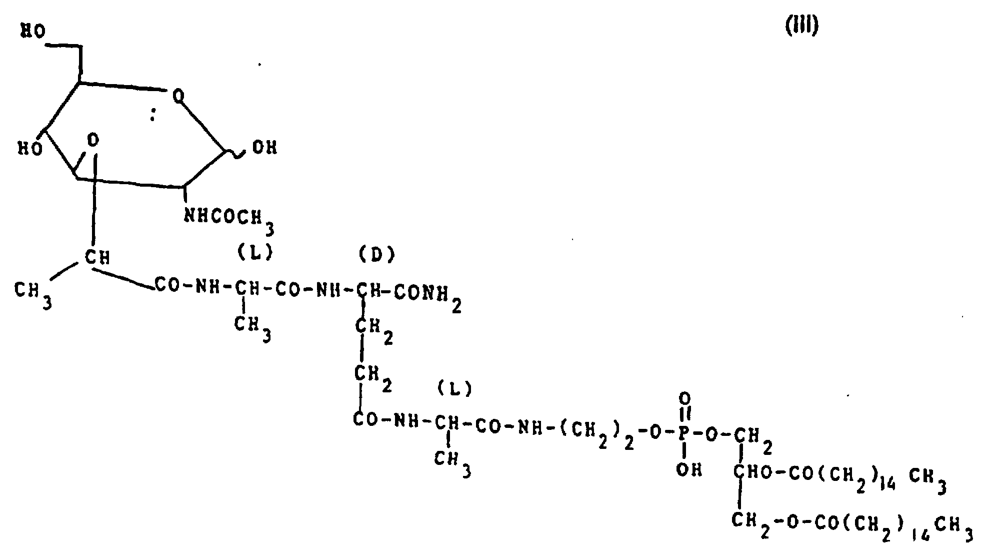

Un exemple particulièrement performant de composé répondant à la formule (I) est le Muradimétide de formule :

Nac-Mur-L-Ala-D-Glu[OMe]-OMe.

Cette composition permet de restaurer un taux normal ou supérieur à la normale des éléments figurés du sang, notamment les erythrocytes et les plaquettes.

L'effet recherché de ces composés est obtenu dans une composition où le dosage des composés de formule (I), (II) ou (III) est tel qu'il permet une administration par voie orale à des doses de 0,1 à 25 mg par kg de poids corporel chez l'homme ou l'animal ou par voie générale dans laquelle le principe actif est dosé de telle façon qu'il permet une administration de 0,05 à 2,5 mg par kg de poids et par dose.

Les traitements par l'AZT présentent l'inconvénient de la myélotoxicité. De façon surprenante, l'administration conjointe d'un dérivé de formule (I) ou (II) ou (III) avec un dérivé de nucléoside de formule générale (IV) ci-dessous conduit à une augmentation d'un facteur 10 à 300, et souvent autour de 200, du taux de CSH dans le sang et les compositions de l'invention contiennent de façon avantageuse, outre un dérivé de formule (I), (II), (III), un dérivé de formule (IV). 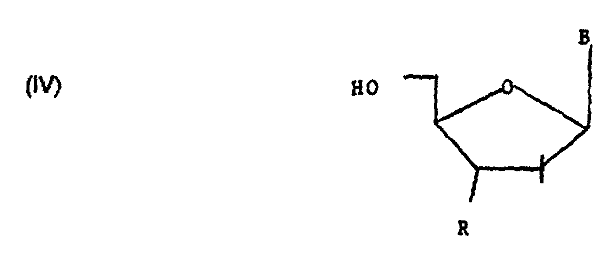 B étant une base purique ou pyrimidique
R = H, N₃, ou un halogène,

Un exemple de ce dérivé, est le 3'-Azido-3'-desoxy-thymidine (AZT). L'association peut également se faire avec tous les nucleosides présentant des activités myélotoxiques et anti-rétrovirales similaires à celle de l'AZT et notamment des nucléosides contenant du ribose et non du désoxyribose. Aussi souvent que nous parlerons dans la suite de cette description de l'AZT, l'homme du métier saura que dans des utilisations similaires d'autres nucléosides peuvent être utilisés et être considérés comme équivalents de l'AZT.

Quand l'AZT est associé au dérivé du MDP dans une composition de l'invention, cette dernière en contient une quantité permettant son administration à une dose comprise entre 1 et 150 mg/kg par voie orale ou par voie générale, cette dose journalière pouvant être administrée en une fois ou fractionnée.

L'administration simultanée de dérivés de MDP de formule (I) (II) ou (III) avec un dérivé de formule (IV) peut être réalisée:
- soit au sein d'un médicament contenant au moins ces deux composés, un tel médicament faisant partie de l'invention,
- soit par l'administration simultanée de deux médicaments dont chacun contient un type de composé, administration simultanée signifiant que les composés peuvent exercer leurs effets pendant au moins une période de recouvrement,
- soit par la réalisation de conjugués associant dans une même molécule les composés de formule (I) et (IV).

Par administration simultanée il est bien entendu que l'administration peut être faite dans une même formulation par voie générale ou par voie orale ou au contraire dans deux formulations différentes et administrées dans un intervalle de temps suffisament réduit pour que les effets de l'un ou l'autre principe actif se fasse sentir simultanément.

L'invention est également relative à des conjugués présentant un effet antimyélotoxique ou permettant de mobiliser les cellules souches hématopoïétiques dans le sang circulant caractérisé par l'une des formules suivantes : 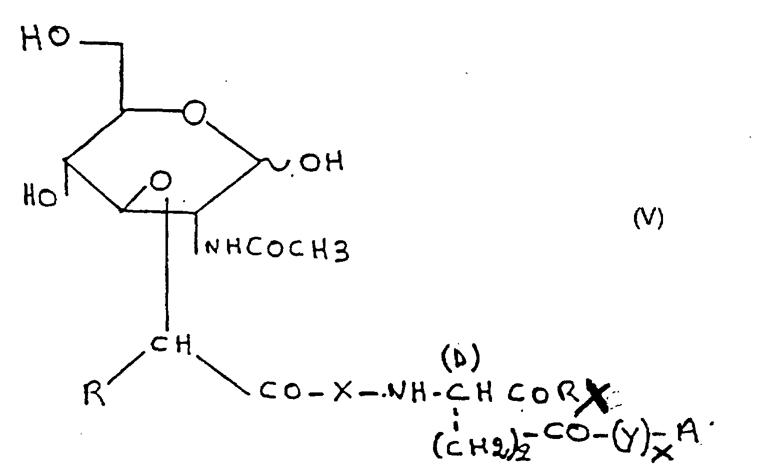 avec
R = H ou CH₃
Rx = NH₂ ou O(CH₂)ₓH avec x = 1 à 4
X = L-Ala ou L-Thr
x = 0, 1 ou 2
Y = un résidu d'acide aminé
avec A étant un nucléoside comportant, en position 5', du résidu désoxyribose soit une fonction hydroxyle, soit une fonction amine, et en position 3' un hydrogène, un halogène ou un groupement azide.
Un conjugué avantageux répondant à la formule (V) est celui où :
R = CH3
X = L-Ala
x=1
Y = L-Ala
Rx = OCH₃
Un autre conjugué de l'invention répond à la formule : 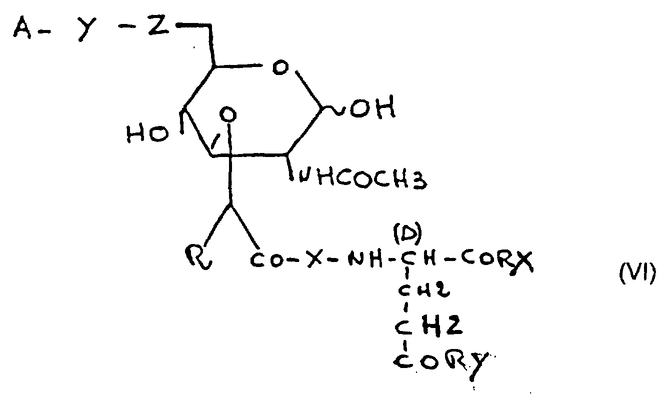 dans lequel
X = L-Ala ou L-Thr
Y est un bras, tel qu'un acide dicarboxylique notamment un résidu succinyle,
Z = O ou NH,
R = H ou CH₃

Rx et Ry ont la signification donnée dans la formule (I), avec A étant un nucléoside comportant, en position 5', du résidu désoxyribose soit une fonction hydroxyle, soit une fonction amine, et en position 3' un hydrogène, un halogène ou un groupement azide.

Un conjugué avantageux répondant à la formule (VI) est celui où :
X = L-Ala
R = CH₃
Rx = OCH₃ et Ry = OCH₃
Y = CO-(CH2)₂-CO
Z = O
A est l'AZT.

La présente invention est également relative aux compositions pharmaceutiques utilisables comme antagonistes des effets myélotoxiques de certains traitements ou médicaments caractérisée en ce qu'elle contient comme principe actif un conjugué de formule V ou VI en association avec un véhicule pharmaceutique acceptable pour une administration per os ou pour une administration par voie générale.

Ces composés sont nouveaux et leurs procédés de synthèses font partie de l'invention.

Pour synthétiser les composés de formule (V), les fonctions amines ou hydroxyles existant éventuellement dans le résidu de base purique ou pyrimidique d'un 2'-désoxyribonucléoside sont temporairement masquées par des groupements de protection temporaire ; la fonction en position 5' du dérivé nucléosidique peut être éventuellement transformée en fonction amine par l'intermédiaire d'un groupement mesyle modificable en un azide finalement hydrogénable en fonction amine; la fonction en position 3' du dérivé nucléoside peut être soit un hydrogène, un halogène ou un groupe azide, soit un groupe mesyle ultérieurement remplacable par des nucléophiles tels que azide ou halogène.

La fonction hydroxyle ou amine en position 5' du dérivé nucléosidique est couplée notamment par une méthode aux anhydrides mixtes ou bien au carbodiimide en utilisant éventuellement certains additifs tels que l'imidazole, l'hydroxybenzotriazole ou encore le DMAP (Di-methyl-aminopyridine), à un dérivé de Muramyl-peptide de formule (I) dans laquelle Ry est un hydroxyle ou un résidu d'acide aminé.

Si Ry est un résidu d'acide aminé, il est possibe d'effectuer d'abord le couplage du dérivé nucléosidique avec l'acide aminé protégé, puis le couplage après déprotection, à un dérivé muramyl-peptide de formule (I) dans laquelle la fonction γ-carboxyle du résidu glutamyle est libre.

En fin de procédure, pour obtenir les conjugués de type (V), les groupements de protection temporaires qui peuvent exister sont éliminés, et, éventuellement, le groupement mesyl en position 3' transformé en un azide ou un halogène.

Une variante consiste à coupler (i) la fonction hydroxyle ou amine en position 5' du dérivé nucléosidique avec un acide aminé protégé, puis par étapes successives, selon des techniques utilisées en synthèse peptidique, (ii) un dérivé de l'acide D-glutamique convenablement protégé, (iii) un dérivé protégé de la L-alanine ou de la L-thréonine, (iv) un dérivé protégé de l'acide N acétylmuramique. En fin de procédure, pour obtenir les conjugués de type (V), les groupements de protection temporaires sont finalement éliminés et, éventuellement, le groupement mesyle en position 3' transformé en un azide ou en un halogène.

Pour synthétiser un composé de formule (VI), les fonctions amines ou hydroxyles existant éventuellement dans le résidu de base purique ou pyrimidique d'un 2'-désoxyribonucléoside sont temporairement masquées par des groupements de protection temporaire ; la fonction en position 5' du dérivé nucléosidique peut être éventuellement transformée en fonction amine par l'intermédiaire d'un groupement mesyle modifiable en un azide finalement hydrogénable en fonction amine.

La fonction en position 3' du dérivé nucléoside peut être soit un hydrogène, un halogène ou un groupe azide, soit un groupe mesyle ultérieurement remplacable par des nucléophiles tels que azide ou halogène.

La fonction hydroxyle ou amine en position 5' du dérivé nucléosidique est couplée notamment par une méthode aux anhydrides mixtes ou bien au carbodiimide en utilisant éventuellement certains additifs tels que l'imidazole, l'hydroxybenzotriazole ou encore le DMAP, à un dérivé Muramyl-peptide de formule (VI) dans laquelle A étant inexistant:
l'hydroxyle anomérique (C1) est ou non protégé temporairement,
Z est NH ou O,
Y est un résidu acide di-carboxylique, notamment un résidu succinyle.

De tels dérivés Muramyl-peptides sont préparés à partir de dérivés Muramyl-peptides de formule (I), dont le mode de synthèse est désormais bien connu, avec l'hydroxyle anomérique (C1) protégé temporairement par un benzyl glycoside hydrogénolysable ou tout autre groupement adéquat, et les hydroxyles en position 4 et 5 protégés par des groupements benzylidènes hydrogénolysables ou isopropylidènes acidolysables. L'élimination de cette protection temporaire permet soit de libérer la fonction hydroxyle primaire (C6) et de la substituer par un groupe succinyle par exemple, soit de la transformer en fonction amine par l'intermédiaire d'un groupement mesyle modifiable en azide finalement hydrogénable en fonction amine. Cette fonction amine est alors substituée par un groupe succinyle par exemple.

En fin de procédure pour aboutir aux conjugués de type (VI), les groupements de protection temporaires qui peuvent exister sont éliminés et, éventuellement, le groupement mesyle en position 3' transformé en un azide ou un halogène.

La présente invention est également relative à l'utilisation d'un composé de formule (I), (II), (III) (V) ou (VI) dans la fabrication d'un médicament antagoniste des effets myélotoxiques de certains traitements ou médicaments ou permettant la stimulation de la fonction hématopoïétique dans la circulation générale seul ou en association avec un composé de formule (IV).

Une telle utilisation est particulièrement recommandée pour contrecarrer les effets myélotoxiques de certaines chimiothérapies pour traiter le sida ou certains cancers.

Par association on entend que les deux composés sont administrés soit dans la même composition pharmaceutique, soit dans une composition différente, les deux compositions étant alors administrées à l'homme ou à l'animal dans un intervalle de temps tel que au moins une partie des effets de l'un et de l'autre composant se produisent simultanément.

Une telle utilisation permet au médicament en question d'enrichir le sang circulant en cellules souches hématopoïétiques ; cet enrichissement est particulièrement avantageux soit lorsque l'on souhaite pratiquer des greffes de moelle osseuse, soit des autogreffes, soit des allogreffes, ou bien encore pour utiliser les CSH pour pratiquer des thérapies géniques, car comme il a été expliqué plus haut les cellules totipotentes que sont les CSH sont les meilleurs candidats pour une bonne expression d'un gène hétérologue après réinjection au patient par voie générale. Il est alors nécessaire de disposer à un temps donné de très fortes concentrations de CHS dans le sang ; il est alors opportun de donner pendant un intervalle de temps court des doses d'AZT supérieures aux doses habituelles qui sont préconisées pour des administrations répétées sur de longues périodes ; il est à noter en outre que les doses d'AZT administrées sont similaires et se situent dans les mêmes gammes que l'administration soit par voie parentérale, par voie orale ou par voie générale

Cette utilisation est caractérisée en ce que le composé de formule (I) est le Muradimétide de formule

Nac-Mur-L-Ala-D-Glu[OMe]-OMe,

ou en ce que le dérivé de formule (II) est la muroctasine ou en ce que le dérivé de formule (III) est le MTP-PE.

Le composé de formule (IV) est la 3'-Azido-3'-desoxy-thymidine (AZT) dans une utilisation préférée de l'invention.

L'invention porte enfin sur les méthodes de traitement par les compositions de l'invention afin de compenser les effets myélotoxiques de certaines chimiothérapies, ou encore d'augmenter la concentration de CHS dans le sang.

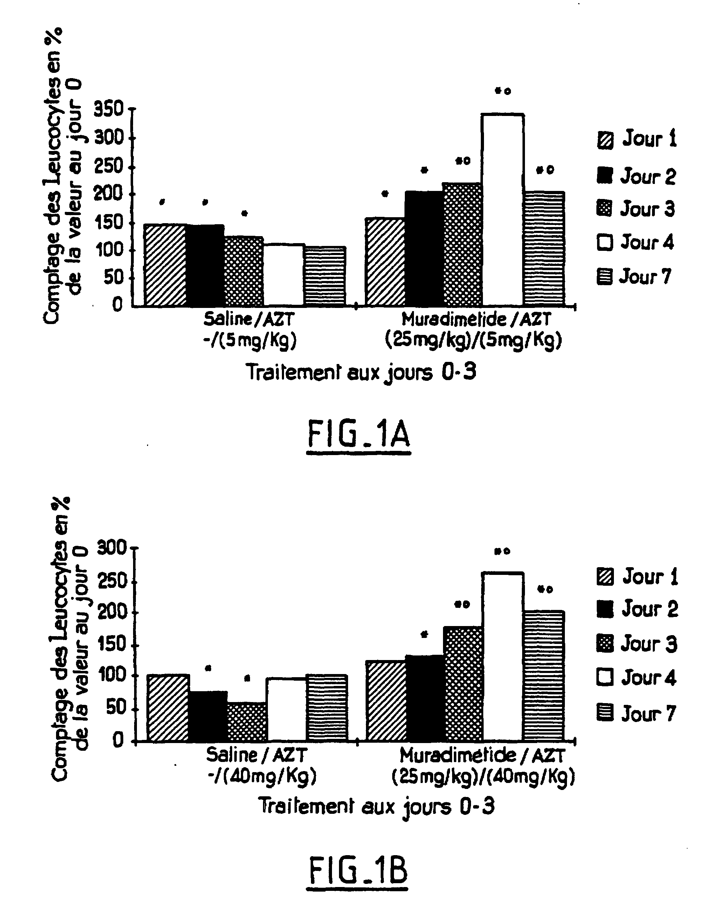

Les exemples suivants sans caractère limitatif sont destinés à montrer l'effet tout à fait surprenant d'un des composés de la famille dans leur nouvelle utilisation. Ils sont effectués chez la souris, et illustrés par les tableaux 1, 2 et 3 qui sont placés dans le texte et par les figures 1 à 8 dont la signification est décrite ci-dessous, illustrent sans ambiguïté l'effet particulier d'un des composants de la classe couverte par la formule (I) à savoir le Muradimétide, à la fois sur l'effet antimyélotoxique et sur la mobilisation des CSH dans la circulation sanguine, cet effet n'étant pas retrouvé chez d'autres dérivés de MDP comme le MDPA ni par des lymphokines utilisées habituellement pour stimuler les myélocytes tel le GM- CSF.

La figure 1 montre l'effet d'un traitement per os des souris avec du Muradimétide en association avec des doses d'AZT non myélotoxiques (5 mg par kg) (Fig 1A) ou myélotoxiques (40mg par kg) (Fig 1B) sur le changement des leucocytes circulants.

La figure 2 représente le même type de courbe sur la numération des neutrophiles ; la figure 2A représente une expérience réalisée avec une dose non myélotoxique d'AZT (5 mg par kg) et la figure 2B avec une dose myélotoxique d'AZT (40 mg par kg).

La figure 3 représente le même type de courbe sur la numération des myélocytes. La figure 3A se rapporte à une dose non myélotoxique d'AZT (5mg/kg) et la figure 3B à une dose myélotoxique (40 mg/kg).

La figure 4 représente les effets comparés du Muradimétide et du rmGM-CSF (recombinant mouse GM-CSF) sur la numération des neutrophiles circulants après administration d'AZT.

La figure 5 représente la même comparaison sur la numération des leucocytes circulants.

La figure 6 représente la même comparaison sur la numération de monocytes circulants.

La figure 7 représente la même comparaison sur la numération des myélocytes circulants.

La figure 8 représente l'effet d'un traitement per os des souris avec du Muradimétide sur le changement de la numération myélocyte dans le sang circulant induite suite à une administration intra-veineuse d'AZT, deux dérivés de MDP étant comparés : le MDPA (Nac-Mur-L-ALa-D-Glu) et le Muradimétide.

La figure 9 représente l'effet du traitement per os de souris BALB/c pendant 7 jours consécutifs avec de l'AZT, du Muradimétide (MDM) ou un mélange de ceux-ci sur la numération plaquettaire.

La figure 10 représente l'effet du traitement per os de souris BALB/c pendant 7 jours consécutifs avec de l'AZT, du Muradimétide (MDM) ou un mélange de ceux-ci sur le nombre des globules rouges.

La figure 11 représente l'effet d'un traitement per os de souris pendant deux semaines consécutives (4 jours par semaine) avec l'AZT, le Muradimétide ou le mélange des deux sur le nombre des myélocytes circulants.

La figure 12 représente la même expérience que celle représentée dans la figure 11 sur des souris ayant subi une ablation de la rate.

La figure 13 représente une expérience similaire sur des souris normales qui ont préalablement reçu un traitement par une drogue cytotoxique : la cyclophosphamide.

### Exemple 1 - Activité du Muradimétide administré seul

Le tableau 1 ci-dessous représente les numérations des leucocytes, neutrophiles, lymphocytes et myélocytes en fonction du temps après traitement par le Muradimétide seul administré à la dose de 25 mg/kg. Le traitement est effectué pendant 4 jours consécutifs par injection intraveineuse.

### Tableau 1

Effet de 4 jours de traitement consécutifs des souris par voie veineuse avec le Muradimétide (25 mg/kg) sur les modifications du nombre de leucocytes circulants normaux (majoritairement neutrophiles et lymphocytes) et immatures (myélocytes).

| Jour de la mesure | Nbre de Leucocytes (x 10⁵/ml) | Nbre de Neutrophiles (x 10⁵/ml) | Nbre de Lymphocytes (x 10⁵/ml) | Nbre de Myélocytes (x 10⁴/ml) |
|---|---|---|---|---|
| 0 | 50 ± 14° | 11±3 | 38±12 | 7 ± 6 |
| 2 | 52±12 | 10±3 | 41 ± 17 | 8±8 |
| 3 | 52 ± 17 | 13 ± 5 | 35 ± 13 | 40±5* |
| 4 | 52 ± 15 | 12 ± 4 | 34 ± 8 | 72 ± 27* |
| 7 | 50±13 | 12±4 | 38±10 | 4 ± 4 |

| | | | | |
|---|---|---|---|---|
| ° Moyenne ± déviation standard (3 souris par groupe). | | | | |
| * Significativement plus élevée que les valeurs au jour 0. | | | | |

Ce tableau indique clairement que le Muradimétide administré seul induit aux jours 3 et 4 une augmentation du nombre de myélocytes circulants qui est hautement significative.

Exemple 2 - Effet d'un traitement par le Muradimétide sur l'augmentation du nombre de neutrophiles, leucocytes et myélocytes après un traitement par l'AZT.

Le traitement par l'AZT seul induit classiquement une diminution du nombre des leucocytes et des neutrophiles circulants. Cette diminution est suivie par une reconstitution intervenant environ 4 à 7 jours après le début du traitement. Quand le Muradimétide a été administré par voie générale ou par la voie orale après un traitement par 40 mg/kg d'AZT, il n'y a pas de baisse du nombre de cellules et au contraire il y a une multiplication par au moins 1,5 du nombre de leucocytes et un triplement de celui des neutrophiles et cela dès après 4 jours. Les résultats sont représentés dans le tableau 2 ci-dessous.

Des souris CDI mâles (5 par groupe) ont été injectées avec de l'AZT à la dose de 40 mg par kg aux jours 0 à 3. Le Muradimétide est administré les mêmes jours à la dose de 10 mg par kg en intraveineux ou de 50 mg par kg per os. Les comptages cellulaires sont réalisés au jour 0 (avant le traitement), et aux jours 3 et 4.

**Tableau 2**

| : Effet du traitement des souris par le Muradimétide par voie veineuse ou per os sur les modifications des comptages cellulaires induites par administration d'AZT (40 mg/kg) par voie veineuse : | | | | |
|---|---|---|---|---|
| Traitement (jours 0-3) | Jour de la mesure | Leucocytes (x 10⁶/ml) | Neutrophiles (x 10⁶ /ml) | Myelocytes (x 10⁴ /ml) |
| eau physiologique | 0 | 5.9 ± 1.5* | 1.5 ± 0.4 | 14 ± 8 |
| Muradimétide i.v; | | 6.0 ± 3.4 | 2.0 ± 1.3 | 5 ± 7 |
| (10mg/kg) | | (NS)° | (NS) | (NS) |
| Muradimétide p.o | | 4.8 ± 0.4 | 1.4 ± 0.5 | 14 ± 13 |
| (50 mg/Kg) | | (NS) | (NS) | (NS) |
| eau physiologique | 3 | 4.2 ± 0.5* | 1.0 ± 0.2 | 2 ± 4 |
| Muradimétide i.v; | | 5.9 ± 2.9 | 1.5 ± 0.8 | 145 ± 83 |
| (10mg/kg) | | (NS)° | (NS) | (0.0143)) |
| Muradimétide p.o | | 6.1 ± 1.4 | 1.6 ± 0.5 | 123 ± 55 |
| (50 mg/Kg) | | (NS) | (NS) | (0.0090)) |
| eau physiologique | 4 | 5.3 ± 1.0* | 1.3 ± 0.5 | 33 ±14 |
| Muradimétide i.v; | | 7.6 ± 1.6 | 4.6 ±1.5 | 117 ± 46 |
| (10mg/kg) | | (0.0143)° | (0.0143)) | (NS) |
| Muradimétide p.o | | 8.1 ± 2.9 | 4.3 ± 2.47 | 138 ± 59 |
| (50 mg/Kg) | | (NS) | (0.0472) | (0.0090) |

| | | | | |
|---|---|---|---|---|
| * moyenne ± déviation standard (5 souris par groupe) | | | | |
| ° pas significativement différent du groupe traité par l'eau physiologique | | | | |

Le tableau 2 montre l'augmentation spectaculaire du pourcentage de myélocytes ou de promyélocytes en présence de Muradimétide quel qu'en soit le mode d'administration.

### Exemple 3 - Effet du traitement combiné per os par l'AZT et le Muradimétide sur les globules rouges et les plaquettes.

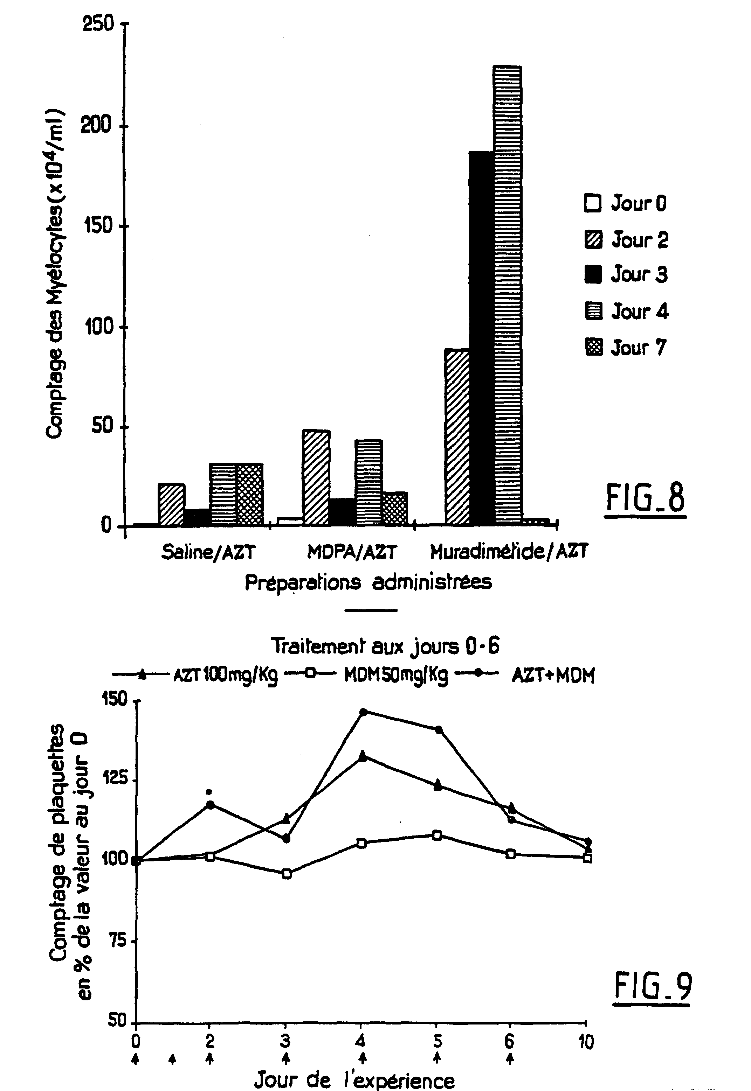

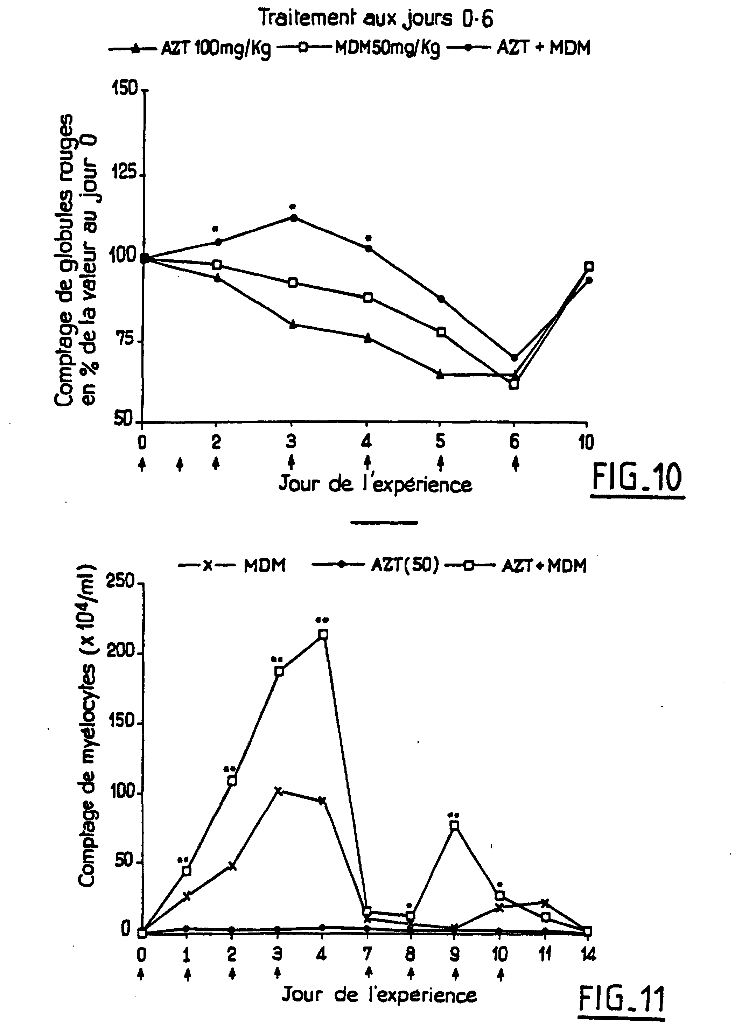

L'effet d'un traitement de 7 jours sur le nombre de globules rouges et de plaquettes est indiqué dans les figures 9 et 10 respectivement. Les numérations sont représentées en pourcentage de celles obtenues au jour 0. Des souris femelles BALB/c (5 par groupe) sont traitées une fois par jour pendant 7 jours consécutifs par 100 mg/kg d'AZT (Δ), 50 mg/kg de muradimétide (D) ou simultanément par 100 mg/kg d'AZT et 50 mg/kg de muradimétide (O). Les comptages de plaquettes ou de globules rouges ont été réalisés au jour 0 et jusqu'à 10 jours suivant le début du traitement. Aucune toxicité sur les plaquettes n'apparaît, que ce soit avec l'AZT, le muradimétide ou le mélange de ceux-ci. La tendance à l'augmentation de leur nombre apparaît entre les jours 4 et 5. En revanche, l'anémie qui pourrait être observée dans les groupes traités pourrait être due aux saignements répétés ; néanmoins la vitesse et l'importance de l'anémie est grandement réduite chez les animaux traités avec le mélange des deux produits en comparaison avec celui observé dans le groupe traité par l'AZT seul. Cela suggère un effet potentiel du mélange pour compenser la toxicité de l'AZT non seulement sur les leucocytes mais également sur les globules rouges.

### Exemple 4 - Mobilisation des CSH après un traitement combiné per os par l'AZT et le Muradimétide.

Le tableau 3 ci-dessous et la figure 11 indiquent l'effet d'un traitement per os des souris avec du Muradimétide avant, en même temps ou après l'AZT administré à 50 mg par kg, sur le nombre de myélocytes circulants testés 24 heures après 4 administrations quotidiennes.

**Tableau 3**

| Temps du traitement p.o. par | | | | |
|---|---|---|---|---|
| AZT (50mg/Kg) | Muradimetide (25mg/Kg) | n = | Nbre de myélocytes circulants (x 10⁴/ml) | P-Value |
| 0 h | - | 5 | 6 ± 8^{Δ} | - |
| - | Oh | 5 | 17 ± 15 | NS |
| 0 h | 0 h | 5 | 215 ± 51 | 0.0090 |
| 0 h | -4 h | 5 | 194 ± 50 | 0.0090 |
| -4 h | 0 h | 5 | 187 ± 42 | 0.0090 |

Le nombre moyen de myélocytes circulant avant le traitement se situe entre 3 et 15 X 10⁴ cellules par ml dans les 5 groupes.

La « P-value » a été calculée par le test Mann Whitney U Rank.

NS signifie que la différence n'est pas significative. Ce tableau confirme que l'augmentation des myélocytes circulants est d'environ 200 quand il y a effet conjoint de l'AZT et du Muradimétide. Les deux constituants de la combinaison ont été administrés per os soit simultanément soit à des temps séparés.

La figure 11 montre l'effet d'un traitement par le MDM à 25 mg/kg (X), de l'AZT à 50 mg/kg (O) et du mélange des deux (□) sur le comptage des myélocytes de souris traitées pendant 2 semaines aux jours 0, 1, 2, 3 de la première semaine et 7, 8, 9, 10 de la deuxième semaine. Il apparaît clairement que le muradimétide seul et surtout le mélange de muradimétide et d'AZT ont un effet significatif sur la mobilisation des CHS alors que l'AZT seul n'a strictement aucun effet. Si l'effet est surtout marqué au niveau de la première semaine, cela montre que le traitement nécessite un certain espace de temps entre les différents cycles de traitement et que des traitements répétés pourraient, le cas échéant, être utiles.

### Exemple 5 - Effet d'un traitement per os d'AZT, de MDM et d'un mélange de ceux-ci sur le comptage de myolécytes circulants de souris splénectomisées.

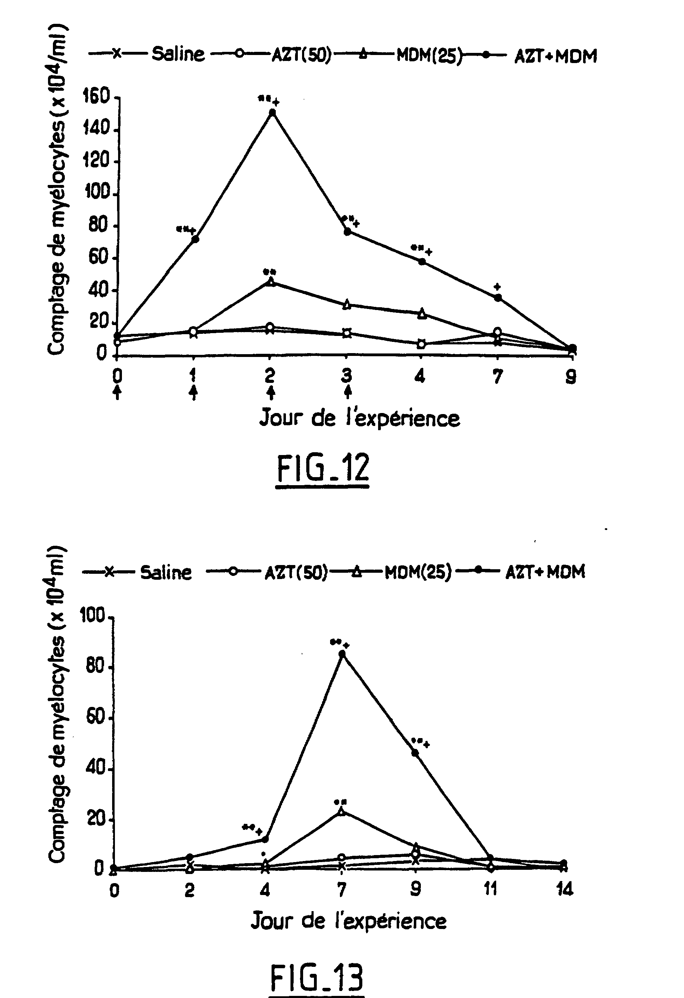

Il est connu chez la souris qu'en sus de la moelle le seul organe ayant une activité hématopoïétique est la rate. Ainsi pour déterminer si l'effet du mélange MDM + AZT sur la mobilisation des CHS provient de la rate ou de la moelle, de l'AZT à 50 mg/kg (O), du MDM à 25 mg/kg (Δ) et le mélange de ceux-ci (O) ont été administrés chez des souris qui avaient été splénectomisées 10 jours plus tôt. Les résultats obtenus sont représentées dans la figure 12 où il est évident que l'administration du mélange pendant 4 jours successifs conduit à une apparition très nette des myélocytes circulants dans la circulation périphérique, et ce avec une intensité similaire à celle observée chez des souris normales (figure 11) et l'augmentation des CHS apparaît dès la deuxième injection. Ceci implique clairement que cette mobilisation des CHS provient de la moelle et non pas de la rate ce qui est extrêmement important pour la transposition d'un tel traitement chez l'homme chez qui c'est la moelle osseuse qui est très majoritairement responsable de la fonction hématopoïétique.

### Exemple 6 - Effet de l'administration orale d'AZT, MDM ou le mélange de ceux-ci sur la mobilisation des CSH de souris traitées par le cyclophosphamide.

L'effet du traitement ci-dessus pour compenser l'effet cytotoxique des chimiothérapies a été étudié en traitant les souris par le cyclophosphamide.

Les souris traitées par une simple injection sous-cutanée de cyclophosphamide (dose de 200 mg/kg) au jour 0 ont été ensuite divisées entre 4 groupes et ont reçu oralement, outre de l'eau physiologique (X), de l'AZT à 50 mg/kg (□), du MDM à 25 mg/kg (Δ) ou du mélange de ceux-ci (O). Le traitement oral a été effectué aux jours 0, 1, 2 et 3 et les numérations cellulaires ont été évaluées jusqu'à 14 jours après le commencement du traitement. Les résultats sont indiqués dans la figure 13. Là-encore, il apparaît évident qu'une très forte mobilisation a lieu surtout en présence du mélange d'AZT et de MDM. Un effet similaire mais nettement moins marqué du MDM administré seul a également été observé sur la numération des neutrophiles circulants.

### Exemple 7 - Effet des compositions de l'invention sur la mobilisation des CSH selon leurs différents stades de différenciation.

Ces expériences ont pour objet la mise en évidence des différentes populations de CSH présentes dans le sang circulant.

Ont été recherchés les stades suivants depuis les plus primitifs décelables en culture et qui ne présentent aucun signe de différenciation comme les CFAC (Coblestone Area Forming Cells) et LTC-IC (Long Term Culture - Initiating Cells) jusqu'au GM-CFU (Granulocyte Macrophage - Colony Forming Units) en passant par des stades intermédiaires comme les HPP et LPP-CFU (respectivement High Proliferating Potential and Low Proliferating Potential CFU). Dans tous les cas, les conditions de cultures, en particulier leur durée, ont été choisies pour permettre une détection optimale des différentes populations considérées.

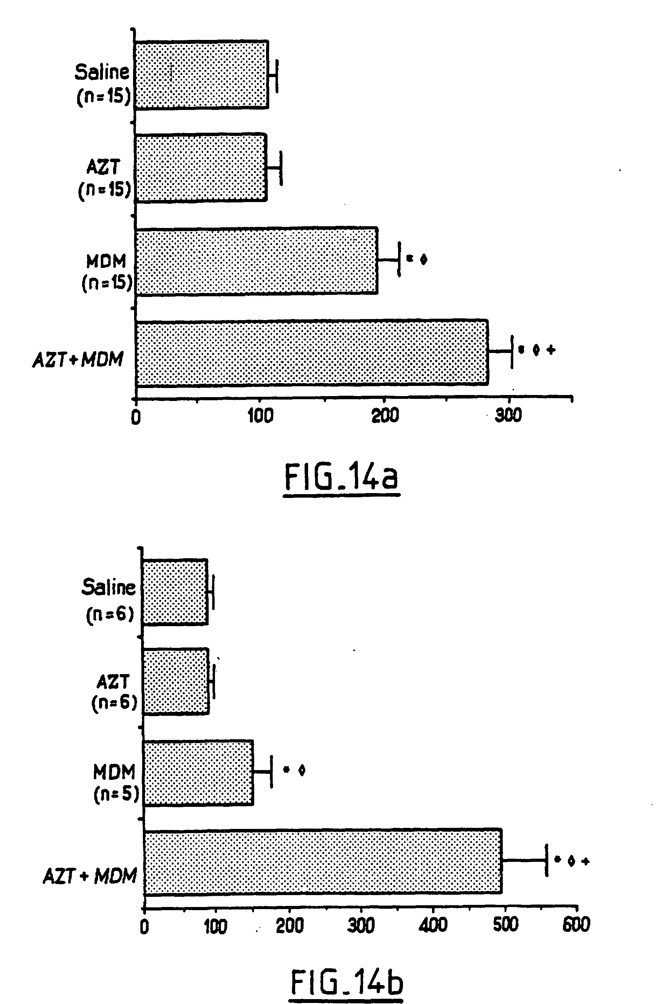

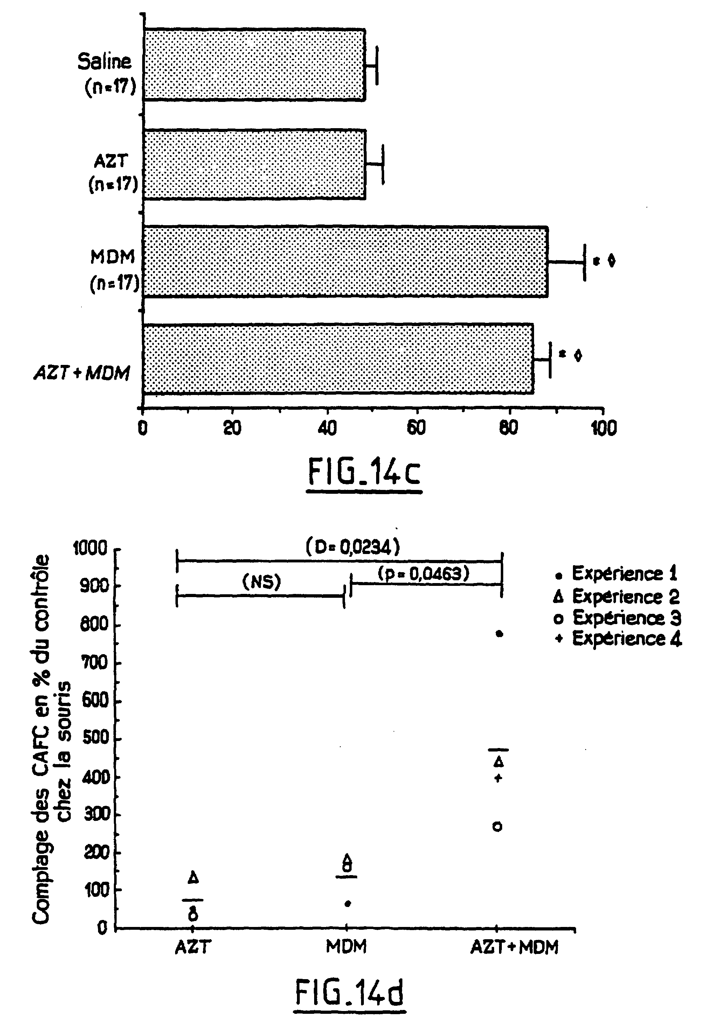

Ont été comparés pour cette étude l'effet de l'injection de saline (X-), d'AZT à 50 mg/kg, du MDM à 25 mg/kg et le mélange des deux sur le nombre de CSH cirulants. Les cellules mononucléaires après purification ont été cultivées pendant 5 jours pour dénombrer les GM-UFC (pour granulocytes - macrophages unités formant colonies), pendant 28 jours pour dénombrer les unités formant colonies à bas potentiel prolifératif (LPP) et les UFC à haut potentiel prolifératif (HPP). Les CAFC ont été dénombrés après 14 jours de culture. Les LTC-IC après 28 jours. Les résultats sont présentés sur les figures 14a, 14b, 14c et 14d respectivement. Les souris (10 Balb/c par groupe) ont reçu une fois par jour et pendant trois jours consécutifs, soit de l'eau physiologique, soit de l'AZT, soit du MDM ou le mélange des deux dans les conditions de doses ci-dessus. Vingt-quatres heures après la dernière administration, le sang de toutes les souris dans chaque groupe a été collecté. Pour les expériences représentées sur les figures 14a, 14b et 14c, les cellules mononucléaires ont ensuite été séparées sur gradients de densité et cultivées dans un milieu semi-solide pendant une période de cinq jours pour les GM-CFU et de 28 jours pour les LPP-CFU. Dans toutes ces courbes, l'effet du MDM seul et surtout du mélange de MDM et d'AZT apparaît clairement.
Pour mesurer l'effet du traitement sur les CAFC et les LTC-IC, le sang a été collecté 24 heures après la demière administration de produit puis les cellules mononucléaires ont été purifiées et cultivées à différentes densités cellulaires sur une couche nourricière de cellules irradiées. Le comptage a été réalisé 14 jours apèrs la mise en culture et est présenté comme un pourcentage de nombre de cellules obtenues dans les cellules contrôles traitées par de l'eau physiologique. les chiffres présentent la moyenne de 3 ou 4 expériences indépendantes. Lorsque la culture est poursuivie pendant 28 jours, des cellules des LTC-IC (pour Long Term Culture Initiating Cells) se forment et l'effet des traitements sur la formation de ces cellules après 28 jours de traitement est similaire à celui observé dans le cas du CAFC.

Toutes ces expériences montrent l'effet particulièrement performant du traitement simultané d'AZT et du MDM, par voie orale, sur la mobilisation des CSH à tous les stades de différenciation.

### Exemple 8 - Effet d'un traitement per os des souris avec du Muradimétide en association avec des doses non myélotoxiques ou myélotoxiques d'AZT sur les changements des comptes de leucocytes et de neutrophiles circulants.

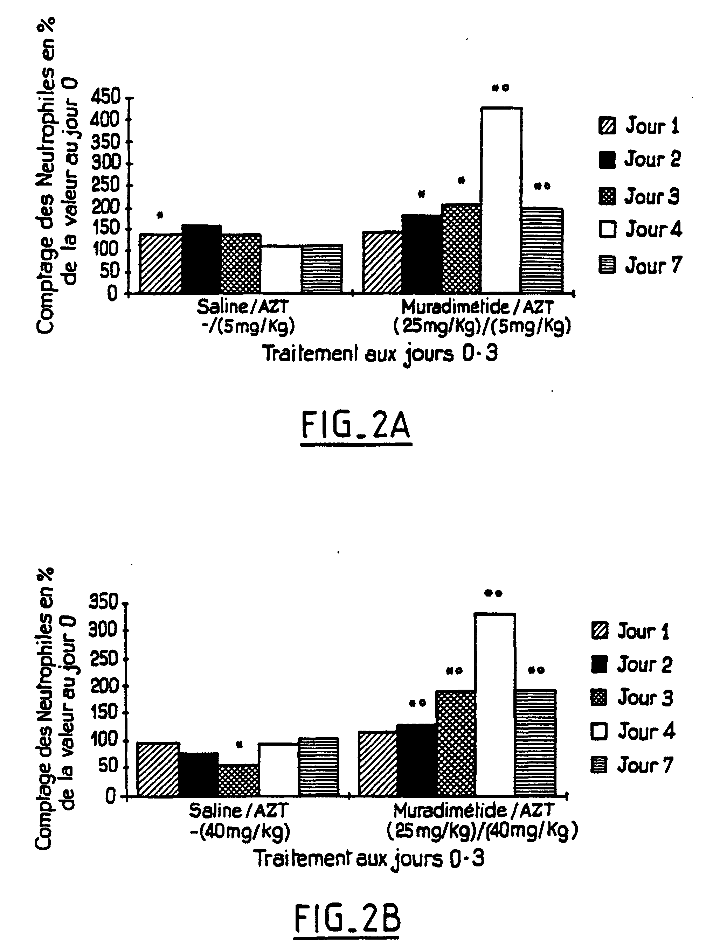

Cet effet est indiqué sur les figures 1 et 2, les figures 1A et 2A représentant une dose non myélotoxique d'AZT et les figures 1B et 2B représentant une dose myélotoxique d'AZT.

Dans cette expérience, des souris Swiss femelles ont été injectées par voie intra-veineuse avec de l'AZT à la dose de 5 ou de 40 mg par kg aux jours 0, 1, 2 et 3. Sur chacun des 4 jours et 4 heures avant l'administration d'AZT, des groupes de souris constitués de 5 individus ont reçu per os soit du tampon soit du Muradimétide à la dose de 25 mg par kg.

Les comptages cellulaires sont effectués aux jours 0, 1, 2, 3, 4 et 7. Les étoiles au-dessus des histogrammes indiquent que les résultats obtenus sont significativement différents des niveaux observés avant le traitement

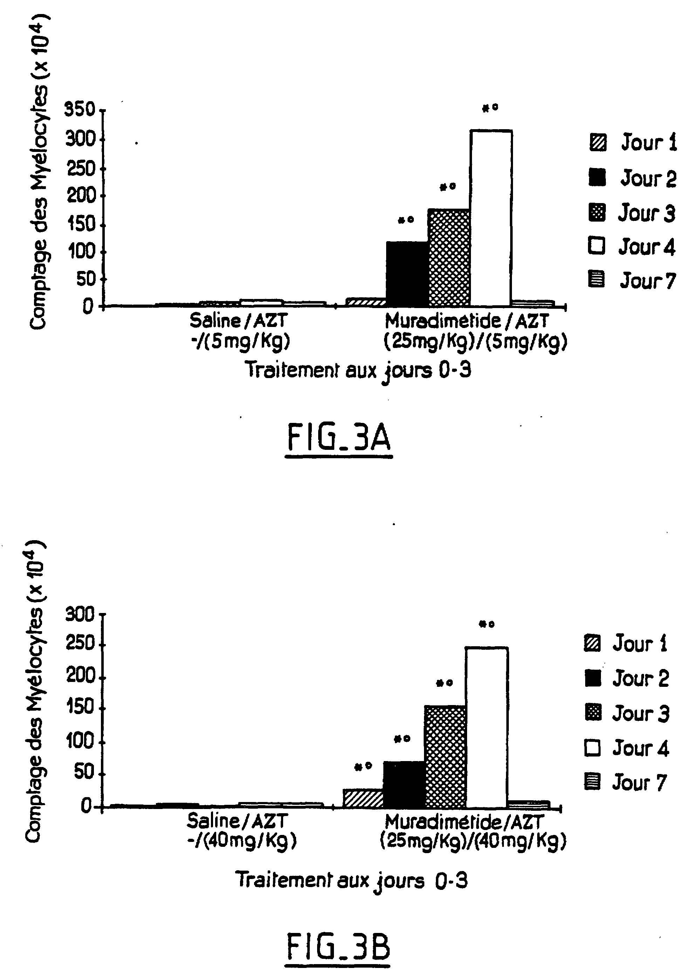

La figure 3 représente la même expérience dans laquelle les myélocytes sont comptés.

Dans ces trois figures 1, 2 et 3, il est clair que l'effet est maximum 4 jours après le début du traitement. Ceci s'explique aisément par le fait que les leucocytes et les neutrophiles ont tendance après cette stimulation à retrouver leur niveau normal, et pour ce qui concerne les myélocytes, le retour au niveau de base signifie que cette stimulation artificielle est tout à fait intéressante pour les cas cités ci-dessus (thérapie génique, greffe de moelle, autogreffe, etc... ). La disparition de cet effet est normale du fait que les CSH se différencient en cellules matures et/ou s'installent dans les organes.

### Exemple 9 - Comparaison des effets du Muradimétide et du GM-CSF.

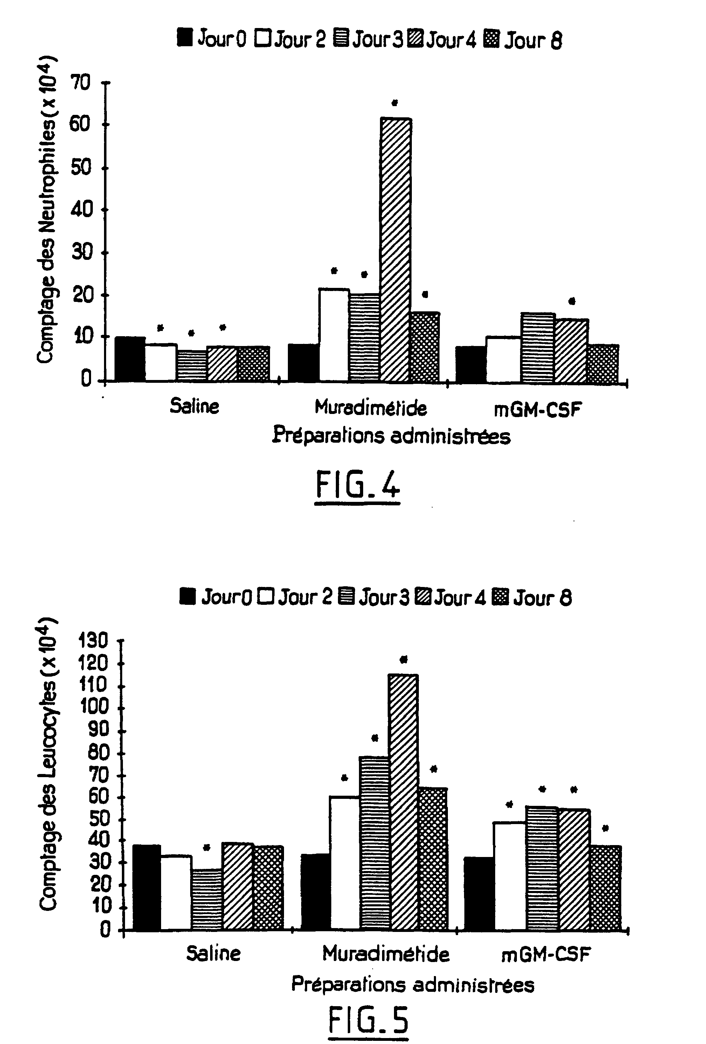

Lorsque les souris ayant reçu un traitement par l'AZT sont traitées par du GM-SCF dans les mêmes conditions que celles utilisées pour le Muradimétide, les comptages de leucocytes et de neutrophiles ne présentent pas d'augmentation contrairement à ce qui est observé lorsque la combinaison AZT-Muradimétide est utilisée. Par contre, on observe une augmentation du nombre des monocytes. Les figures 4, 5, 6, et 7 indiquent l'effet d'un traitement des souris avec du Muradimétide ou avec du mGM-CSF sur les changements respectivement dans les neutrophiles circulants, dans les leucocytes circulants ou dans les monocytes circulants ou dans les myélocytes circulants après une administration intraveineuse d'AZT.

Les souris Swiss femelles ont été traitées par voie intra-veineuse avec 40 mg par kg d'AZT aux jours 0, 1, 2 et 3. Les groupes de souris (5 par groupe) reçoivent 4 heures avant l'AZT soit de l'eau physiologique, soit du Muradimétide à 25 mg par kg ou du mGM-CSF à 125 µg par kg par voie intraveineuse. Les comptages sont réalisés aux jours 0, 2, 3, 4 et 8. Les étoiles au-dessus des histogrammes indiquent qu'il existe une différence significative du comptage au regard des valeurs obtenues avant le traitement au jour 0. On voit donc que si le mGM-CSF est capable d'entraîner une augmentation du nombre de monocytes, le Muradimétide a un effet nettement marqué sur les neutrophiles et les leucocytes. De plus le Muradimétide contrairement au GM-CSF induit une très forte élévation des CSH dans le sang.

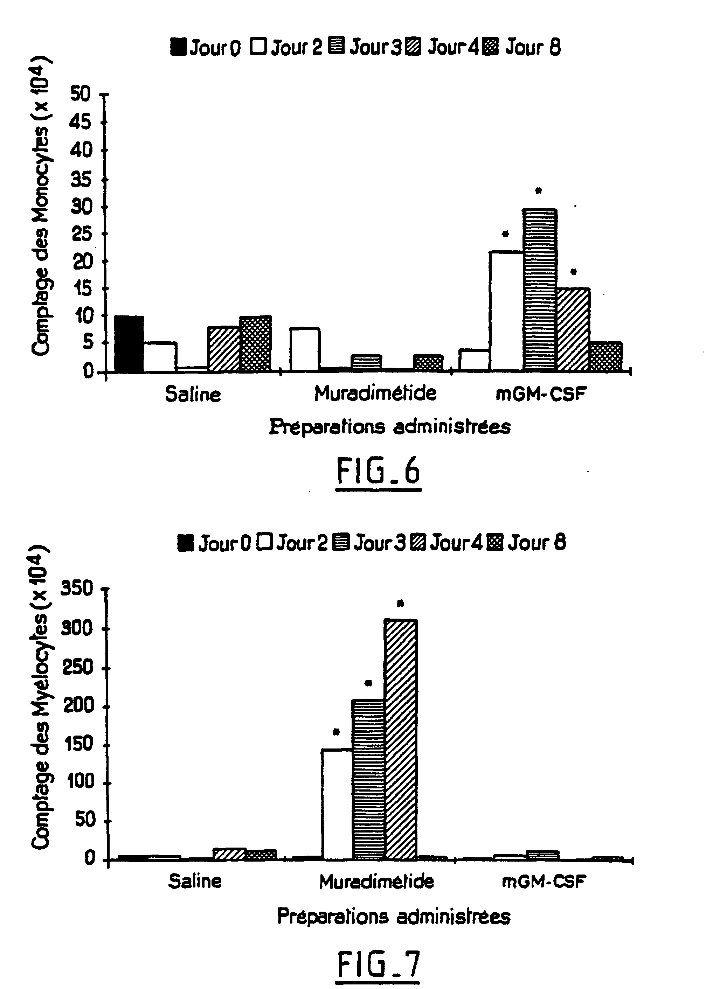

L'effet du Muradimétide comparé à celui du mGM-CSF est explicite sur la figure 7 qui montre clairement que le Muradimétide mobilise des CSH dans des conditions où le GM-CSF ne le fait pas.

### Exemple 10 - Comparaison de l'effet du Muradimétide avec un autre dérivé du MDP.

Le dérivé utilisé pour mettre en évidence l'effet particulier du Muradimétide est le MDPA. La figure 8 indique l'effet sur des souris d'un traitement per os sur les changements de la numération de myélocyte induite suite à une administration intraveineuse d'AZT. Dans cette expérience, les souris swiss femelles ont été traitées de façon intraveineuse avec 40 mg par kg d'AZT aux jours 0, 1, 2 et 3. Des groupes de 5 souris ont reçu aux mêmes quatre jours et 4 heures avant l'AZT soit de l'eau physiologique, soit du MDPA à la dose de 25 mg par kg ou du Muradimétide par voie orale à la dose de 25 mg par kg. Les comptages de myélocytes ont été réalisés aux jours 0, 2, 3, 4 et 8. Les étoiles au-dessus des histogrammes ont la même signification que dans les diagrammes précédents.

Il apparaît clairement que la propriété des dérivés de formule (I) et en particulier du Muradimétide n'est pas une propriété commune à tous les muramyl peptides. Ceci est particulièrement bien démontré par la comparaison avec le MDPA dont le Muradimétide est le dérivé diester.

### Exemple 11 - Synthèse de N-acetyl-muramyl-L-alanyl-D-glutaminique α-méthyl ester γ-L-alanine 3'azido-3'-deoxythymidine (formule V).

Cette synthèse comprend les étapes suivantes :
a) Synthèse de Boc-L-alanine 3'-azido-3'-deoxythymidine (1).
   A 189mg (immole) de Boc-L-alanine dissous dans 20 ml de dimethylformamide anhydre contenant 102 mg (1mmole) de dimethylamino-pyridine sont ajoutés 206 mg (1mmole) de N-N'-dicyclohexylcarbodiimide. 267 mg (1mmol) de 3'-azido-3'-deoxythymidine (AZT) sont ajoutés au mélange réactionnel qui est agité pendant 24 heures à la température ambiante. Après filtration du précipité de dicyclohexylurée, le mélange réactionnel est concentré sous vide. Le produit est finalement obtenu après purification sur colonne de gel de silice α-benzyl-N-acetyl-muramyl-L-alanyl-D-glutamique di-methyl ester.
b) synthèse de L-alanine 3'-azido-3'-deoxythymidine, chlorhydrate (2) :
   A 260 mg (0,6 mmmole) de (1) sont dissous dans 2ml d'une solution 1N d'acide chlorhydrique dans l'acide acétique glacial. Après 30 minutes à température ambiante, le mélange réactionnel est concentré à sec. Le résidu obtenu est repris dans de l'acétone et concentré à sec. Le processus est répété plusieurs fois et le résidu, desséché sous vide, est directement utilisé à l'étape suivante : 225mg (100%).
c) synthèse de N-acetyl-muramyl-L-alanyl-D-glutamique α-méthyl ester γ-L-alanine 3'-azido-3'-deox-thymidine(3) (formule V) :
   A 507 mg (1 mole) de N-acetyl-muramyl-L-alanyl-D-glutamique α-méthyl ester ( ) en solution dans 10 ml de dimethylformamide anhydre sont successivement ajoutés à -15°C 0,11 ml (1 mmole) de N-méthylmorpholine et 0,13 ml (1mmole) de chlorocarbonate d'isobutyle. Après 5 minutes sont ajoutés 225 mg (0,60 mmole) de (2) en solution dans 5 ml de diméthyl formamide refroidie à -15°C, contenant 0,11 ml de N-méthylmorpholine. Le mélange réactionnel est agité pendant 24 heures à -15°c puis concentré sous vide. Le produit est finalement obtenu après purification sur colonne de Lichroprep RP18 (Merck) par élution dans un mélange d'éthanol et d'eau. (3) est finalement obtenu par lyophilisation de sa solution aqueuse : 272 mg (60%).

### Exemple 12 - Synthèse de 6-0-(Succinyl-3'-azido-3'-deoxythymidine)-N-acetyl-muramyl-L-alanyl-D-glutamique diméthyl ester (formule VI).

a) synthèse de α-Benzyl-N-acetyl-muramyl-L-alanyl-D-glutamique di-methyl ester (1) :
   1,4 g (2 mmole) de α-benzyl-4,6-OBzi-N-acetyl-muramyl-L-alanyl-D-glutamique di-methyl ester (.) sont traités à reflux en présence de 100 ml d'acide acétique aqueux à 560% jusqu'à dissolution complète. Le mélange réactionnel est concentré sous vide. Le résidu obtenu est repris et concentré successivement dans de l'eau, du toluène et du méthanol, puis désséché sous vide. (1) est purifié sur colonne de gel de silice par élution avec un mélange de chloroforme et de méthanol : 305 mg (50%).
b) Synthèse de 6-0-succinyl-N-acetyl-muramyl-L-alanyl-D-glutamique di-methyl ester (2) :
   A 300 mg (1mmole) de (1) dissous dans 10 ml de pyridine anhydre. 44 mg (4 mmoles) d'anhydride succinique. Le mélange réactionnel est agité à température ordinaire pendant 4 jours, puis concentré sous vide. Le résidu obtenu est repris dans l'acétone et concentré plusieurs fois, puis desséché sous vide.
   Le produit obtenu (α-benzyl-6-0-succinyl-N-acetyl-muramyl-L-alanyl-D-glutamique di-methyl ester) est dissous dans 15 ml d'acide acétique glacial et hydrogéné en 4 heures à la pression atmosphérique en présence de 100 mg de Pd 5 % sur charbon. Après filtration du catalyseur, le mélange réactionnel est concentré sous vide. Le résidu obtenu est repris dans l'acétone et concentré plusieurs fois, puis déchessé sous vide. Il est alors repris dans l'eau et purifié sur colonne d'AG1X2 (H+)(BIORAD) par élution avec un gradient en acide acétique aqueux. (2) est finalement obtenu par lyophilisation de sa solution aqueuse : 450 mg (70%).
c) Synthèse de 6-0-(Succinyl-3'-azido-3'-deoxythymidine)-N-acetyl-muramyl-L-alanyl-D-glutamique di-methyl ester (3) (formule VI) :
   A 320 mg (0,5 mmole) de (2) en solution dans 10 ml de dimethylformamide anhydre sont successivement ajoutés à -15°C 0,05 ml (0,5 mmole) de N-méthylmorpholine et 0,06 ml (0,5 mmole) de chlorocarbonate d'isobutyle. Après 5 minutes sont ajoutés 267 mg (1 mmole) de 3'-azido-3'deoxythymidine (AZT). Le mélange réactionnel est agité pendant 24 heures à 15°C puis concentré sous vide. Le produit est finalement obtenu après purification sur colonne de Lichroprep RP18 (Merck) par élution dans un mélange d'éthanol et d'eau. (3) est finalement obtenu par lyophilisation de sa solution aqueuse : 440 mg (50%).

Les composés de formules (I), (II) et (III) d'une part en association ou non avec un dérivé de formule (IV), ou des nouveaux conjugués répondant aux formules (V) et (VI) qui sont en fait des liaisons covalentes des composés de formule (I) avec des composés de formule (IV) offrent une nouvelle classe de médicaments présentant un spectre très large d'utilisation ; outre les exemples cités et revendiqués ils doivent pouvoir être utilisés dès que le rétablissement de la numération formulaire dans le sang est nécessaire ou lorsque pour une raison ou une autre l'accès aux myélocytes via la circulation sanguine est nécessaire. Les CSH sont des cellules totipotentes d'une importance extrême comme il est expliqué au début de cette description. Grâce à cette invention, leur accès par un simple prélèvement sanguin, soit à titre d'outils biologique, soit à titre thérapeutique, devient possible sans manipulations longues et douloureuses.

## Revendications

1. Utilisation d'un composé de formule : 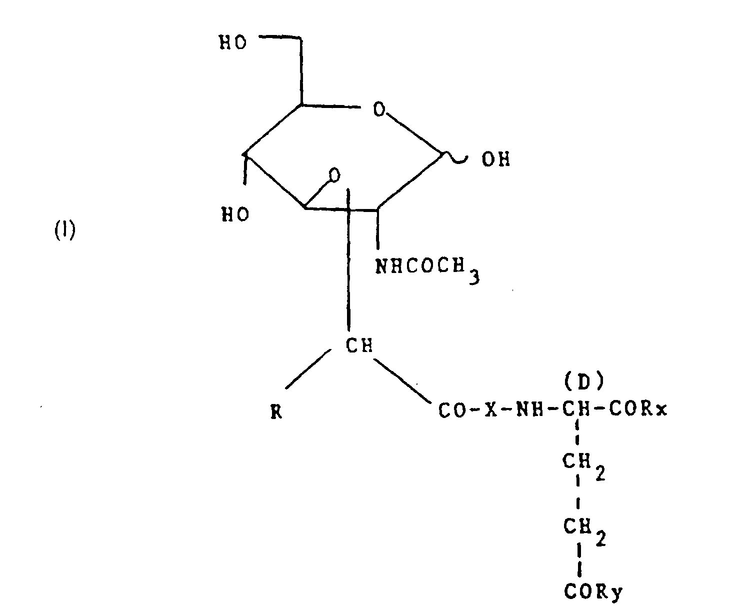 avec
R = H ou CH₃
X = L-ALa ou L-Thr
Rx = NH₂ ou 0(CH₂)ₓH avec x = 1 à 4
Ry = OH (sauf si Rx = NH2) ou
O(CH₂)ₓH avec x = 1 à 4
ou la muroctasine de formule (II) suivante :
Nac-Mur-L-Ala-D-isoGln-N^{Σ} stearoyl-L-Lys, 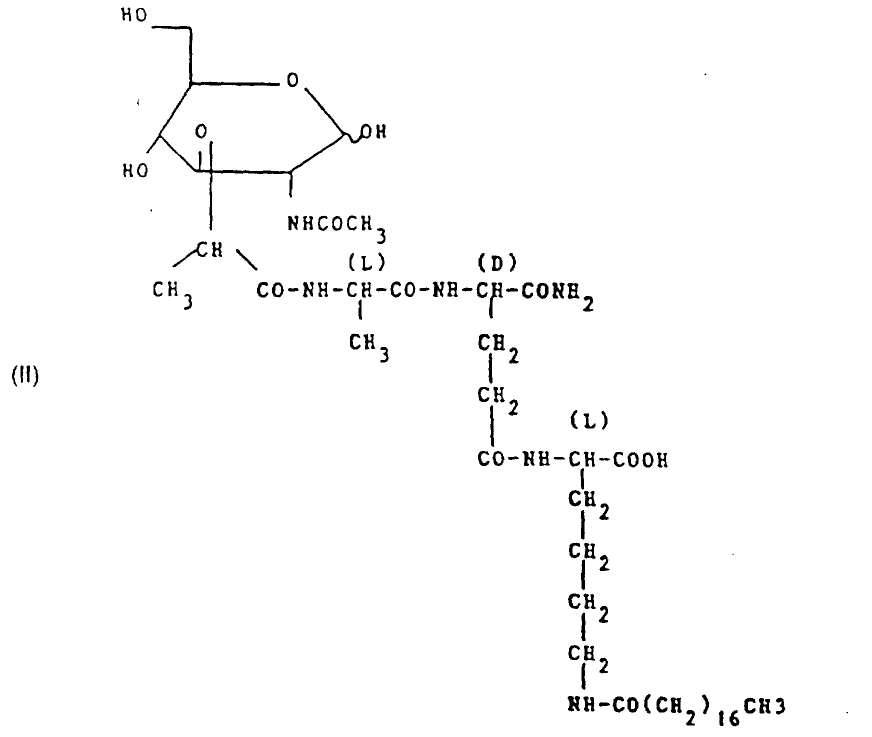 ou le MTP-PE de formule (III) suivante Nac-Mur-L-Ala-D-isoGln-L-alayl-2 (1',2'dipalmitoyl-sn-glycero-3'-phosphoryl) ethylamide mono-sodium 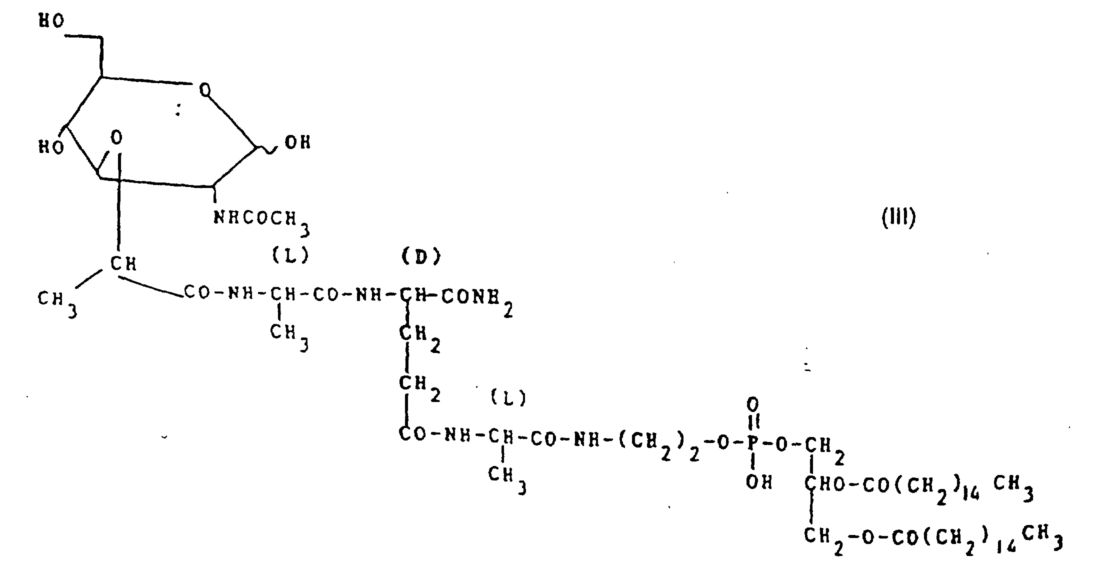 dans un procédé pour la fabrication d'une composition pharmaceutique destinée à induire la stimulation de cellules souches hématopoïétiques et à les mobiliser dans le sang circulant.

2. Utilisation selon la revendication 1, **caractérisée en ce que** le dosage de la composition est de 0,1 à 25 mg par kg de poids corporel.

3. Utilisation selon la revendication 1 ou 2, **caractérisée en ce que** le composé répondant à la formule (I) est le muradimétide de formule Nac-Mur-L-Ala-D-Glu[OMe]-OMe.

4. Utilisation selon l'une des revendications 1 à 3, **caractérisée en ce que** la composition comporte en outre un dérivé de nucléoside de formule générale: 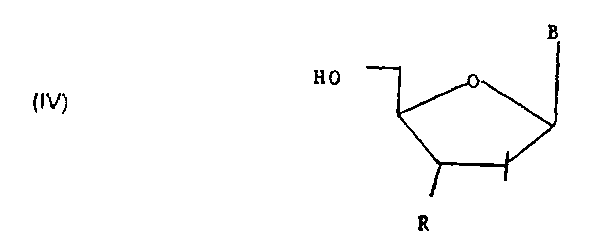 B étant une base purique ou pyrimidique, et
R étant H, N₃ ou un halogène.

5. Utilisation selon la revendication 4, **caractérisée en ce que** le dérivé de nucléoside est la 3'-azido-3'-désoxy-thymidine (AZT).

6. Utilisation selon la revendication 4 ou 5, **caractérisée en ce que** le dosage du dérivé de nucléoside est de 1 à 150 mg/kg.

7. Utilisation selon l'une des revendications 1 à 6, **caractérisée en ce que** l'administration du muradimétide est orale.

8. Utilisation selon l'une des revendications 1 à 7, **caractérisée en ce que** l'administration du muradimétide est réalisée par voie générale.

9. Utilisation selon les revendications 1 à 7, **caractérisée en ce que** l'administration du dérivé de nucléoside est orale.

10. Utilisation selon les revendications 1 à 7, **caractérisée en ce que** l'administration du dérivé de nucléoside est réalisée par voie générale.

11. Utilisation selon la revendication 1 **caractérisée en ce que** le dérivé de formule (III) est le MTP-PE.

12. Utilisation selon la revendication 1 **caractérisée en ce que** le dérivé de formule (II) est la muroctasine.

13. Utilisation d'un conjugué **caractérisé par** sa formule (V): 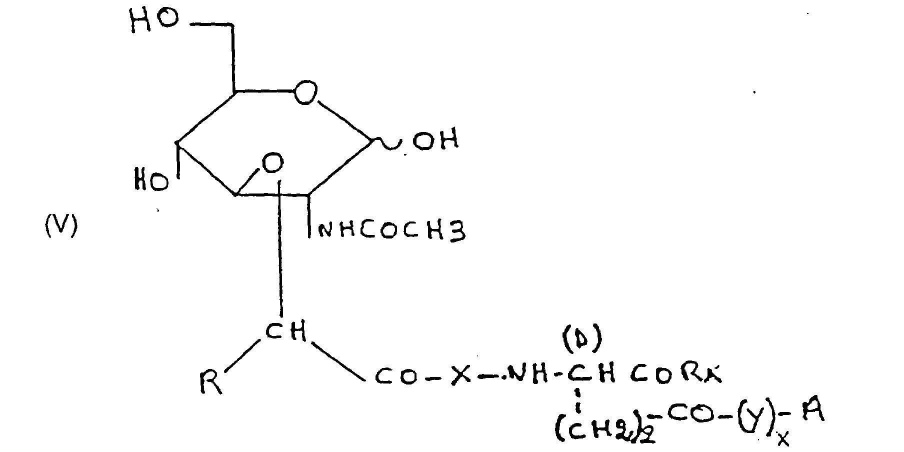 dans laquelle
R = H ou CH₃
Rx = NH₂ ou 0(CH₂)ₓH avec x = 1 à 4
X = L-Ala ou L-Thr
x = 0, 1 ou 2
Y = un résidu d'acide aminé
avec A étant un nucléoside comportant, en position 5', du résidu désoxyribose soit une fonction hydroxyle, soit une fonction amine, et en position 3' un hydrogène, un halogène ou un groupement azide dans un procédé pour la fabrication d'une composition pharmaceutique destinée à induire la stimulation de cellules souches hématopoïétiques et à les mobiliser dans le sang circulant.

14. Utilisation d'un conjugué **caractérisé par** sa formule (VI) : 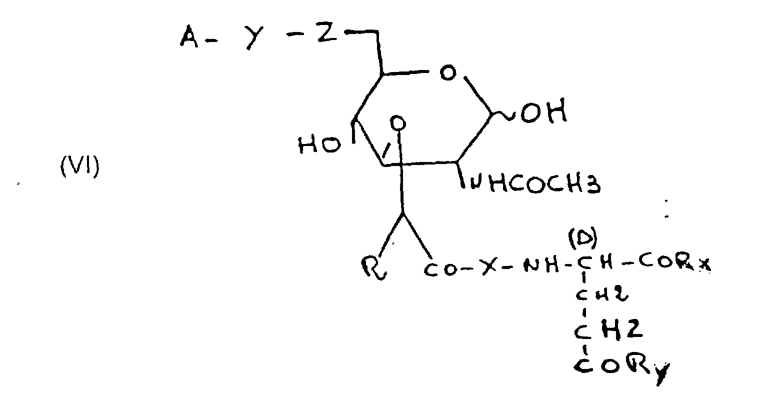 dans laquelle
x = 0 ou 1
X = L-Ala ou L-Thr
Y = est un bras, tel qu'un acide dicarboxylique notamment un résidu succinyle
Z = est O ou NH
R = H ou CH₃
Rx et Ry ont la signification donnée dans la formule (I),
avec A étant un nucléoside comportant, en position 5', du résidu désoxyribose soit une fonction hydroxyle, soit une fonction amine, et en position 3' un hydrogène, un halogène ou un groupement azide dans un procédé pour la fabrication d'une composition pharmaceutique destinée à induire la stimulation de cellules souches hématopoïétiques et à les mobiliser dans le sang circulant.

15. Procédé de synthèse d'un conjugué de formule (V) pour l'utilisation selon l'une des revendications 13 ou 14 **caractérisé, en ce qu'**il comprend au moins les étapes suivantes :
a) synthèse Boc-L-alanine 3'-azido-3'-deoxythymidine (1) à partir de Boc-L-alanine et d'AZT.
b) synthèse de L-alanine 3'-azido-3'-deoxythymidine, chlorhydrate (2) à partir de (1),
c) synthèse de N-acetyl-muramyl-L-alanyl-D-glutamique α-méthyl ester γ-L-alanine 3'-azido-3'-deox-thymidine(3) par addition de de N-acetyl-muramyl-L-alanyl-D-glutamique α-méthyl ester à (2).

16. Procédé de synthèse d'un conjugué de formule (VI) pour l'utilisation selon l'une des revendications 13 ou 14 **caractérisé, en ce qu'**il comprend au moins les étapes suivantes :
a) Obtention de a-benzyl-N-acetyl-muramyl-L-alanyl-D-glutamique di-methyl ester (1) à partir de α-benzyl-4,6-OBzi-N-acetyl-muramyl-L-alanyl-D-glutamique di-methyl ester,
b) Obtention de 6-0-succinyl-N-acetyl-muramyl-L-alanyl-D-glutamique di-methyl ester (2) par succinylation de (I),
c) Synthèse de 6-0-(Succinyl-3'-azido-3'-deoxythymidine)-N-acetyl-muramyl-L-alanyl-D-glutamique di-méthyl ester (3) en couplant à (2) de l'AZT.

## Patentansprüche

1. Verwendung einer Verbindung der Formel: 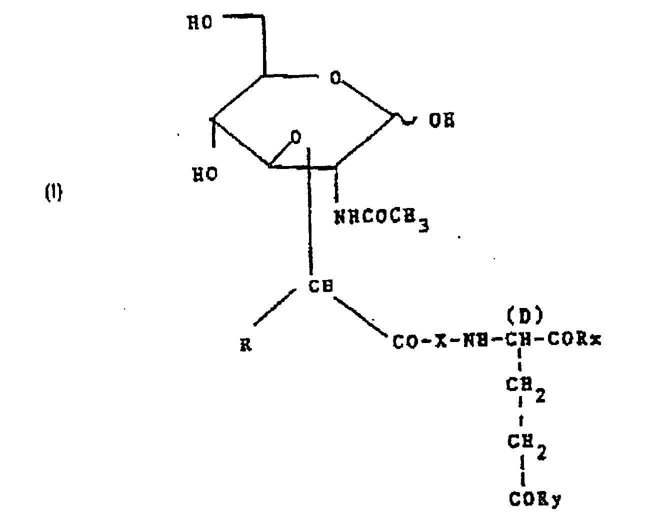 wobei R = H oder CH₃
X = L-Ala oder L-Thr
Rx = NH₂ oder O(CH₂)ₓH, wobei x = 1 bis 4
Ry = OH (außer wenn Rx = NH₂) oder
O(CH₂)ₓH, wobei x = 1 bis 4
oder des Muroctasins der folgenden Formel (II):
Nac-Mur-L-Ala-D-isoGln-N^{Σ}-stearoyl-L-Lys 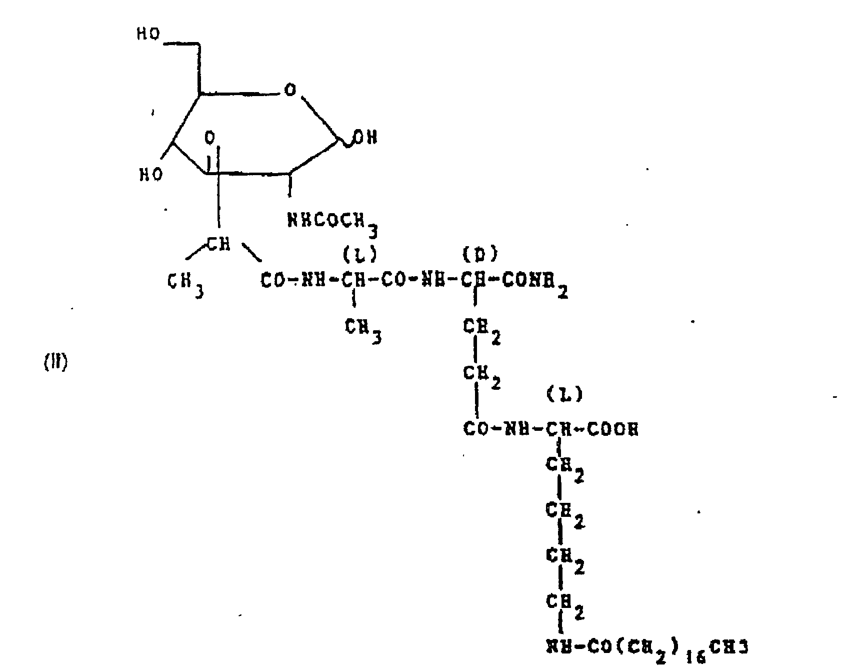 oder des MTP-PE der folgenden Formel (III)
Nac-Mur-L-Ala-D-isoGln-L-alanyl-2-(1',2'-dipalmitoyl-sn-glycero-3'-phosphoryl)ethylamid-Mononatrium 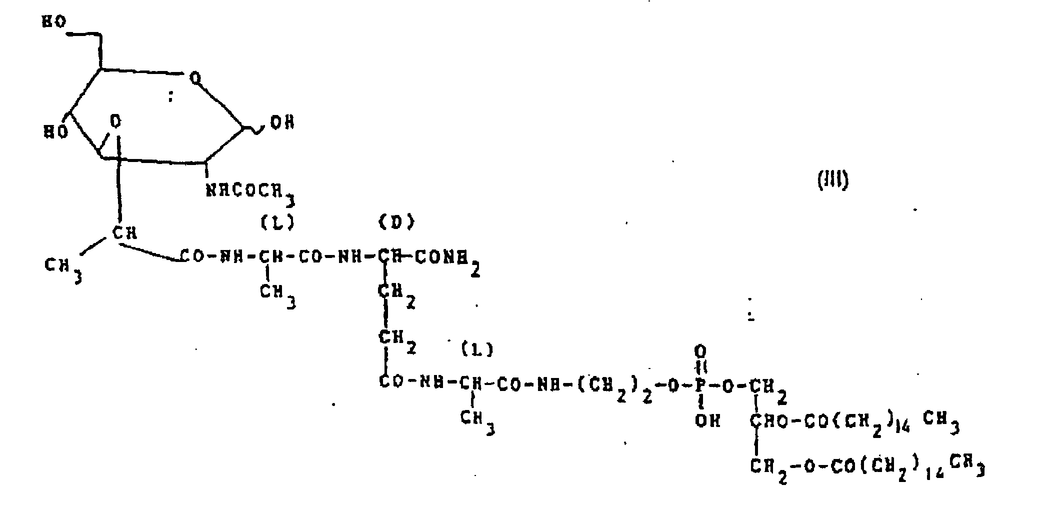 in einem Verfahren zur Herstellung eines Arzneimittels, das dazu bestimmt ist, die Stimulation von hämatopoetischen Stammzellen zu bewirken und diese im Blutkreislauf zu mobilisieren.

2. Verwendung nach Anspruch 1, **dadurch gekennzeichnet, daß** die Dosierung der Zusammensetzung 0,1 bis 25 mg pro kg Körpergewicht beträgt.

3. Verwendung nach Anspruch 1 oder 2, **dadurch gekennzeichnet, daß** die Verbindung nach Formel (I) das Muradimetid der Formel ist:
Nac-Mur-L-Ala-D-Glu[OMe]-OMe.

4. Verwendung nach einem der Ansprüche 1 bis 3, **dadurch gekennzeichnet, daß** die Zusammensetzung weiter ein Nukleosid-Derivat der allgemeinen Formel umfaßt: 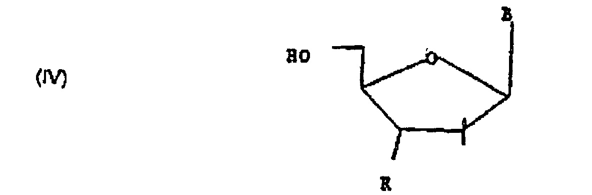 wobei B eine Purin- oder Pyrimidin-Base ist und
R H, N₃ oder ein Halogenatom ist.

5. Verwendung nach Anspruch 4, **dadurch gekennzeichnet, daß** das Nukleosid-Derivat das 3'-Azido-3'-desoxythymidin (AZT) ist.

6. Verwendung nach Anspruch 4 oder 5, **dadurch gekennzeichnet, daß** die Dosierung des Nukleosid-Derivats 1 bis 150 mg/kg beträgt.

7. Verwendung nach einem der Ansprüche 1 bis 6, **dadurch gekennzeichnet, daß** die Verabreichung des Muradimetids oral erfolgt.

8. Verwendung nach einem der Ansprüche 1 bis 7, **dadurch gekennzeichnet, daß** die Verabreichung des Muradimetids auf allgemeinem Wege erfolgt.

9. Verwendung nach den Ansprüchen 1 bis 7, **dadurch gekennzeichnet, daß** die Verabreichung des Nukleosid-Derivats oral erfolgt.

10. Verwendung nach den Ansprüchen 1 bis 7, **dadurch gekennzeichnet, daß** die Verabreichung des Nukleosid-Derivats auf allgemeinem Wege erfolgt.

11. Verwendung nach Anspruch 1, **dadurch gekennzeichnet, daß** das Derivat der Formel (III) MTP-PE ist.

12. Verwendung nach Anspruch 1, **dadurch gekennzeichnet, daß** das Derivat der Formel (II) Muroctasin ist.

13. Verwendung eines Komplexes, **gekennzeichnet durch** seine Formel (V): 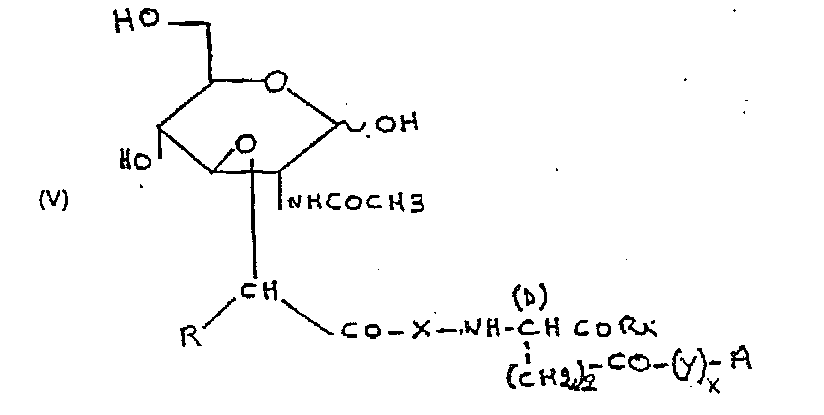 in der
R = H oder CH₃
Rx = NH₂ oder O(CH₂)ₓH, wobei x = 1 bis 4
X = L-Ala oder L-Thr
x = 0, 1 oder 2
Y = ein Aminsäure-Rest
wobei A ein Nukleosid ist, das an Position 5' des Desoxyribose-Rests entweder eine Hydroxylfunktion oder eine Aminfunktion umfaßt und an Position 3' ein Wasserstoffatom, ein Halogenatom oder eine Azidgruppe umfaßt,
in einem Verfahren zur Herstellung eines Arzneimittels, das dazu bestimmt ist, die Stimulation von hämatopoetischen Stammzellen zu bewirken und diese im Blutkreislauf zu mobilisieren.

14. Verwendung eines Komplexes, **gekennzeichnet durch** seine Formel (VI): 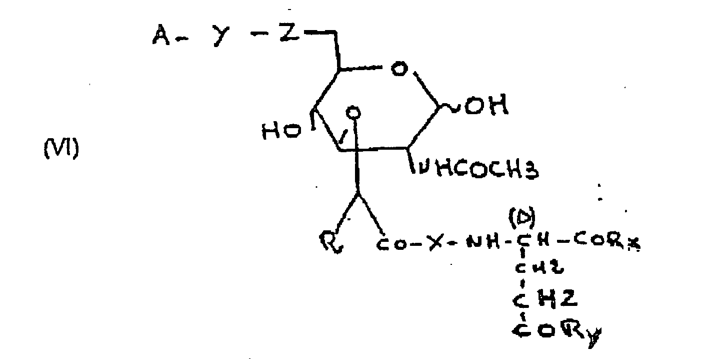 in der
x = 0 oder 1
X = L-Ala oder L-Thr
Y = eine Verzweigung, wie z.B. eine Dicarbonsäure, insbesondere ein Succinyl-Rest
Z = O oder NH
R = H oder CH₃
Rx und Ry die in Formel (I) angegebene Bedeutung haben,
wobei A ein Nukleosid ist, das an Position 5' des Desoxyribose-Rests entweder eine Hydroxylfunktion oder eine Aminfunktion aufweist und an Position 3' ein Wasserstoffatom, ein Halogenatom oder eine Azidgruppe aufweist,
in einem Verfahren zur Herstellung eines Arzneimittels, das dazu bestimmt ist, die Stimulation von Blut-Stammzellen zu bewirken und diese im Blutkreislauf zu mobilisieren.

15. Verfahren zur Synthese eines Komplexes der Formel (V) zur Verwendung nach einem der Ansprüche 13 oder 14, **dadurch gekennzeichnet, daß** es mindestens die folgenden Schritte umfaßt:
a) Synthese von Boc-L-alanin-3'-azido-3'-desoxythymidin (1) ausgehend von Boc-L-alanin und AZT,
b) Synthese von L-Alanin-3'-azido-3'-desoxythymidin, Chlorhydrat (2) ausgehend von (1),
c) Synthese von N-Acetylmuramyl-L-alanyl-D-glutamin-a-methylester-y-L-alanin-3'-azido-3'-desoxythymidin (3) durch Zugabe von N-Acetylmuramyl-L-alanyl-D-glutamin-α-methylester zu (2).

16. Verfahren zur Synthese eines Komplexes der Formel (VI) zur Verwendung nach einem der Ansprüche 13 oder 14, **dadurch gekennzeichnet, daß** es mindestens die folgenden Schritte umfaßt:
a) Darstellung von α-Benzyl-N-acetylmuramyl-L-alanyl-D-glutamindimethylester (1) ausgehend von α-Benzyl-4,6-OBzi-N-acetylmuramyl-L-alanyl-D-glutamin-dimethylester,
b) Darstellung von 6-O-Succinyl-N-acetylmuramyl-L-alanyl-D-glutamindimethylester (2) durch Succinylierung von (1),
c) Synthese von 6-O-(Succinyl-3'-azido-3'-desoxythymidin)-N-acetylmuramyl-L-alanyl-D-glutamindimethylester (3) durch Kopplung von AZT an (2).

## Claims

1. Use of the compound of formula: 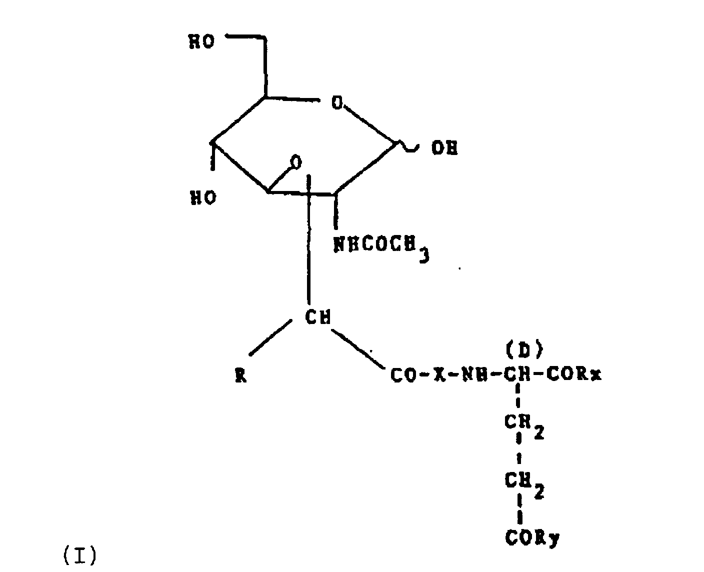 where
R = H or CH₃
X = L-Ala or L-Thr
Rx = NH₂ or O(CH₂)ₓH with x = 1 to 4
Ry = OH (except if Rx = NH₂) or O(CH₂)ₓH with x = 1 to 4
Or the muroctasin of the following formula (II):
Nac-Mur-L-Ala-D-isoGin-N^{Σ} stearoly-L-Lys, 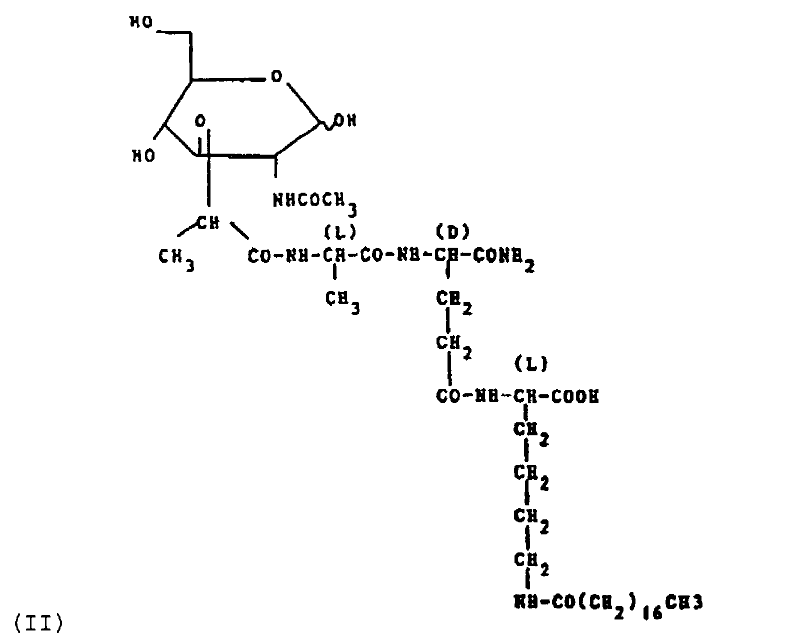 or the MTP-PE of the following formula (III) Nac-Mur-L-Ala-D-isoGln-L-alanyl-2 (1',2' dipalmitoyl-sn-glycero-3'-phoshoryl) ethylamide monosodium 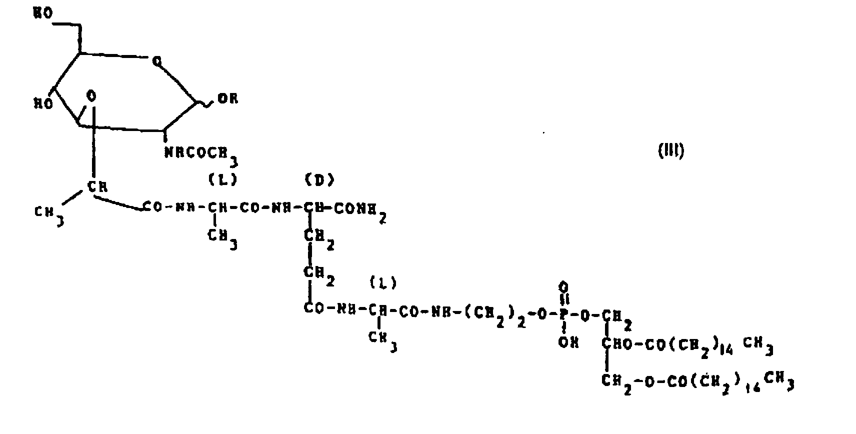 in a method for manufacturing a pharmaceutical compound intended for inducing stimulation of hematopoietic stem cells and for mobilizing the hematopoietic stem cells in the circulating blood.

2. Use according to claim 1, **characterised in that** the dosage of the compound ranges from 0.1 to 25 mg per kg of corporeal weight.

3. Use according to claim 1 or 2, **characterised in that** the compound corresponding to formula (I) is muradimetide of formula:
Nac-Mur-L-Ala-D-Glu[OMe]-OMe.

4. Use according to any of the claims 1 to 3, **characterised in that** the compound comprises moreover a nucleoside derivative of general formula: 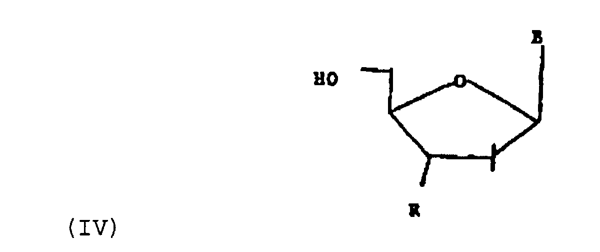 B being a purine or pyrimidic base, and
R being H, N3 or a halogen.

5. Use according to claim 4, **characterised in that** the dosage of the nucleoside derivative is 3'-azido-3'-desoxy-thymidine (AZT).

6. Use according to claim 4 or 5, **characterised in that** the dosage of the nucleoside derivative ranges from 1 to 150 mg/kg.

7. Use according to any of the claim 1 to 6, **characterised in that** muradimetide is administered orally.

8. Use according to any of the claim 1 to 7, **characterised in that** muradimetide is administered sytemically.

9. Use according to any of the claim 1 to 7, **characterised in that** the nucleoside derivative is administered orally.

10. Use according to any of the claim 1 to 7, **characterised in that** the nucleoside derivative is administered by systemically.

11. Use according to claim 1, **characterised in that** the derivative of formula (III) is MTP-PE.

12. Use according to claim 1, **characterised in that** the derivative of formula (II) is muroctasin

13. Use of the conjugate **characterised by** its formula (V): 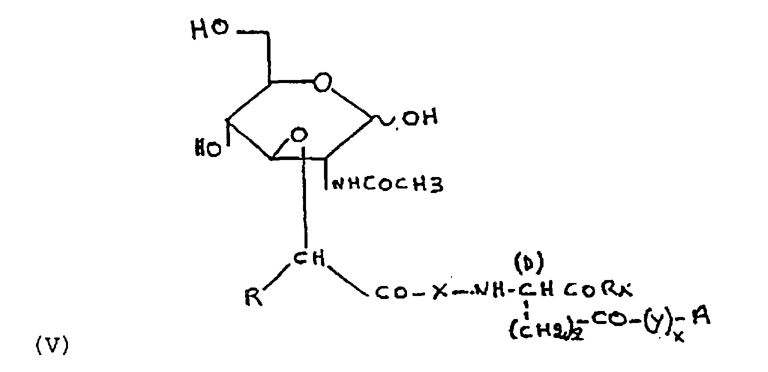 where
R = H or CH₃
Rx = NH₂ or O(CH₂)ₓH with x = 1 to 4
X = L-Ala or L-Thr
x = 0, 1 or 2
Y = an amino acid residue
with A being a nucleoside comprising, in the position 5', a desoxyribose residue, either a hydroxyl function, or an amino function, and in the position 3' a hydrogen, a halogen or an azide grouping in a method for manufacturing a pharmaceutical compound intended for inducing stimulation of hematopoietic stem cells and for mobilizing the hematopoietic stem cells in the circulating blood.

14. Use of the conjugate '**characterised by** its formula (VI): 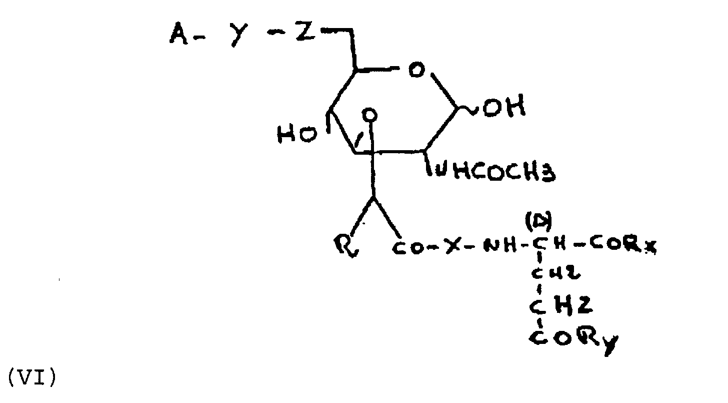 where
x = 0 or 1
X = L-Ala or L-Thr
Y = is an arm, such as dicarboxylic acid, notably a succinyl residue
Z = is O or NH
R = H or CH₃
Rx and Ry have the signification given in formula (I), with A being a nucleoside comprising, in the position 5', a desoxyribose residue, either a hydroxyl function, or an amino function, and in the position 3' a hydrogen, a halogen or an azide grouping in a method for manufacturing a pharmaceutical compound intended for inducing stimulation of hematopoietic stem cells and for mobilising the hematopoietic stem cells in the circulating blood.

15. A method for synthesising a conjugates of formula (V) for an use according to one of the claims 13 or 14, **characterised in that** it comprises at least the following steps:
a) Synthesis of Boc-L-alanine 3'-azido-3'-deoxythymidine (1) from Boc-L-alanine and AZT,
b) Synthesis of L-alanine 3'-azido-3'-deoxythymidine, chlorhydrate (2) from (1)
c) Synthesis of N-acetyl-muramyl-L-alanyl-D-glutamic α-methyl ester Υ-L-alanine 3'-azido-3'-deox-thymidine (3) by addition of N-acetyl-muramyl-L-alanyl-D-glutamic α-methyl ester to (2).

16. A method for synthesising a conjugate of formula (VI) for an use according to one of the claims 13 or 14, **characterised in that** it comprises at least the following steps:
a) preparation of α-benzyl-N-acetyl-muramyl-L-alanyl-D-glutamic di-methyl ester (1) from α-benzyl-4,6-OBzi-N-acetyl-muramyl-L-alanyl-D-glutamic di-methyl ester
b) preparation of 6-0-succinyl-N-acetyl-muramyl-L-alanyl-D-glutamic di-methyl ester (2) by succinylation of (I)
c) synthesis of 6-0-(succinyl-3'-azido-3'-deoxythymidine)-N-acetyl-muramyl-L-alanyl-D-glutamic di-methyl ester (3) by coupling to (2) with AZT.
